# EUROPEAN PATENT APPLICATION

(11) **EP 4 116 282 A1**
(43) Date of publication of application: **11.01.2023**
(21) Application number: 21764165.3
(22) Date of filing: 02.03.2021
(51) Int. Cl.: C07C 1/32, C07C 15/20, C07C 25/22, C07C 41/30, C07C 43/205, C07C 209/68, C07C 211/45, C07D 333/16, C07D 333/18, C07D 333/56, C07C 17/26, C07B 61/00

(54) **METHOD FOR PRODUCING MONO-CROSS-COUPLED AROMATIC COMPOUND HAVING LEAVING GROUP**

(30) Priority: 02.03.2020 JP 2020035093
(71) Applicant: National University Corporation Hokkaido University, Sapporo-shi, Hokkaido 060-0808 (JP)
(72) Inventor: ITO, Hajime, Sapporo-shi, Hokkaido 060-0808 (JP); KUBOTA, Koji, Sapporo-shi, Hokkaido 060-0808 (JP)
(74) Representative: Vossius & Partner Patentanwälte Rechtsanwälte mbB
(86) International application number: PCT/JP2021/007912
(87) International publication number: WO 2021/177290

(57) **Abstract**

Disclosed is a method for producing a mono-cross-coupled aromatic compound (3-1) having one less leaving group than an aromatic compound (1) having at least two leaving groups, the method comprising: preparing the aromatic compound (1) having at least two leaving groups; preparing a compound (2) capable of undergoing a cross-coupling reaction selected from an aromatic boronic acid (2-1), an aromatic amino compound (2-2), a diboronic acid ester (2-3), an aromatic compound (2-4) having a hydroxyl group and an aromatic compound (2-5) having a thiol group; and performing a cross-coupling reaction of the aromatic compound (1) having at least two leaving groups with the compound (2) in the presence of a palladium catalyst and a base, in the absence of a solvent.

## Description

### [Technical Field]

The present invention relates to a novel method for producing a mono-cross-couped (obtained by performing one cross-coupling reaction) aromatic compound having a leaving group without using a solvent. It relates to a method in which selective conversion into aromatic groups is performed from an aromatic compound having a plurality of leaving groups (e.g., an aromatic compound in which a plurality of groups having an aromatic group is bonded to an aromatic ring) while leaving a specific number of leaving groups, thus producing a mono-cross-coupled aromatic compound having a leaving group (e.g., an aromatic compound in which a group having an aromatic group and a leaving group such as halogen are bonded to an aromatic ring). Furthermore, it relates to a method for producing a monohalogenated mono-cross-coupled aromatic compound from a dihalogenated aromatic compound, and also relates to a method for producing a di-cross-coupled (obtained by performing two cross-coupling reactions) aromatic compound using a monohalogenated mono-cross-coupled aromatic compound.

### [Background Art]

Aromatic compounds having a plurality of aromatic groups which are substituted on an aromatic compound have widely been used in the medical and electronics fields. The production of aromatic compounds having a plurality of aromatic groups is generally performed by using an aromatic compound having a plurality of leaving groups such as halogen as a substrate, and converting the leaving groups into aromatic groups. For example, when synthesizing a compound having two different types of aromatic groups in an aromatic ring, one aromatic group is first introduced into an aromatic compound having two leaving groups, and then another type of an aromatic group is introduced.

In organic synthetic chemistry, it is exceedingly difficult to selectively convert only leaving group at a certain position for a molecule having a plurality of the same leaving groups. For example, when the Suzuki-Miyaura cross-coupling reaction is performed on a substrate having two halogeno groups, a mixture of a mono-coupled product and a di-coupled product is generally obtained. In the prior art, the mono-coupled product is selectively synthesized by applying an electronic or steric bias to substrate molecules. However, since this method limits the applicable substrate, new synthetic strategy has been required (see, for example, NPL 1).

The present inventors have made a study on an organic synthesis reaction using a ball mill and reported that the objective compound can be produced in nearly 100% yield in a cross-coupling reaction of a compound in a solid state (NPL 2). However, there is no report of any selectivity in the reaction with regard to the cross-coupling reaction of the compound having a plurality of leaving groups.

### [Citation List]

### [Non-Patent Literature]

[NPL 1] Sewan Theeramunkong, Antonio Caldarelli, Alberto Massarotti, Silvio Aprile, Diego Caprioglio, Roberta Zaninetti, Alessia Teruggi, Tracey Pirali, Giorgio Grosa, Gian Cesare Tron, and Armando A. Genazzani, J. Med. Chem., 2011, Vol.54, 4977-4986
[NPL 2] Koji Kubota, Tamae Seo, Katsumasa Koide, Yasuchika Hasegawa, and Hajime Ito, Nature Communications, 2019, 10: 111

### [Summary of Invention]

### [Technical Problem]

Thus, it is an object of the present invention to establish a method capable of easily producing a mono-cross-coupled aromatic compound having a leaving group (e.g., an aromatic compound in which a group having an aromatic group and a leaving group such as halogen are bonded to an aromatic ring), which is a precursor when producing an aromatic compound having a plurality of aromatic groups (e.g., an aromatic compound in which a plurality of groups having an aromatic group are bonded to an aromatic ring) from an aromatic compound having a plurality of leaving groups. For example, as a typical example, it is an object to provide a method for producing a monohalogenated mono-cross-coupled aromatic compound (a mono-coupled product or a first reaction product) (e.g., an aromatic compound in which a group having an aromatic group and one halogen are bonded to an aromatic ring) from a dihalogenated aromatic compound, more selectively and efficiently. In a conventional method, for example, an electronic or steric bias is applied to a substrate molecule in order to obtain superiority of the reactivity for the substrate having two halogeno groups, which gives a complex structure to the design of the compound as the substrate. It is an object of the present invention to provide a technique capable of producing a monohalogenated mono-cross-coupled aromatic compound in which only one halogen is converted into a substituent having an aromatic group, using, as a substrate, a dihalogenated aromatic compound having a simple structure into which the same type of halogen is introduced.

It is also an object of the present invention to provide a method for producing a di-cross-coupled aromatic compound (di-cross-coupled product), which comprises allowing a mono-cross-coupled aromatic compound to undergo further coupling reaction.

### [Means for Solving the Problems]

The present inventors have made a study on an organic synthesis reaction using a ball mill and found that the reaction is highly dependent on a state of a substrate and a product (hardness or softness of the solid, viscosity of the liquid, or the gas) since an organic solvent is not used in the reaction.

The present inventors have found that, for example, in a palladium-catalyzed cross-coupling reaction using a ball mill, the liquid substrate is more reactive than the solid substrate. Therefore, the present inventors have thought that once a liquid dihalide is converted into a solid mono-coupled product (a monohalide or a first reaction product) by a mechanochemical cross-coupling reaction, the solid mono-coupled product is less likely to be further converted into a di-coupled product (or a second reaction product). That is, they have thought that the dihalide, which is a liquid raw material, reacts preferentially compared to the mono-coupled product and, as a result, the mono-coupled product is selectively obtained.

They have also found that, not only by changes from the solid state to liquid state of the substrate and product, but also by controlling the state of the solid (hardness or softness of the solid), the mono-coupled product (or the first reaction product) can be selectively produced in reference to the di-coupled product (or the second reaction product), thus completing the present invention.

The present invention provides a method for producing a mono-cross-coupled aromatic compound (3-1) having one less leaving group than an aromatic compound (1) having at least two leaving groups, the method comprising:
preparing the aromatic compound (1) having at least two leaving groups;
preparing a compound (2) capable of undergoing a cross-coupling reaction selected from an aromatic boronic acid (2-1), an aromatic amino compound (2-2), a diboronic acid ester (2-3), an aromatic compound (2-4) having a hydroxyl group and an aromatic compound (2-5) having a thiol group; and
performing a cross-coupling reaction of the aromatic compound (1) having at least two leaving groups with the compound (2) in the presence of a palladium catalyst and a base, in the absence of a solvent.

The present invention provides, in another aspect, a method for producing a di-cross-coupled aromatic compound (3-2) which underwent two cross-coupling reactions, the method comprising allowing the above-mentioned mono-cross-coupled aromatic compound (3-1) to undergo a second cross-coupling reaction.

### [Advantageous Effects of Invention]

When using the production method of the present invention, it is possible to produce a mono-cross-coupled aromatic compound having a leaving group, which can be a precursor of an aromatic compound having a plurality of aromatic group, in high selectivity and high yield. The reaction process can be significantly shortened, leading to cost advantage. The organic synthesis reaction using a ball mill uses almost no organic solvent, thus enabling elimination of the problem of disposal of the organic solvent.

When using the production method of the embodiment of the present invention, for example, a monohalogenated mono-cross-coupled aromatic compound (3-1) can be produced from a dihalogenated aromatic compound (1), more selectively and efficiently. Since the dihalogenated aromatic compound is inexpensive and distributed in large quantities, there is no need to use expensive raw materials, thus making it possible to provide a monohalogenated mono-cross-coupled aromatic compound (3-1) at low cost.

Furthermore, in the embodiment of the present invention, the monohalogenated mono-cross-coupled aromatic compound (3-1) can be further cross-coupled to produce, as a di-cross-coupled product (3-2), an aromatic compound having a plurality of aromatic groups which is widely used in the medical and electronics fields.

### [Brief Description of the Drawings]

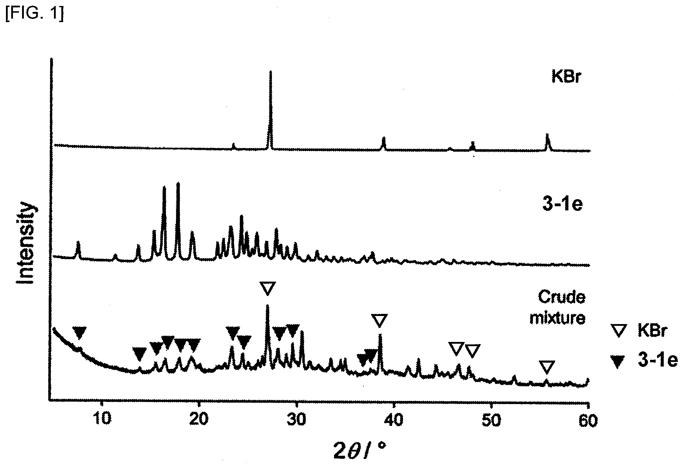

FIG. 1 shows the observation of a solid phase palladium-catalyzed cross-coupling reaction of Example 5 by powder X-ray diffraction.

### [Description of Embodiments]

A method for producing a mono-cross-coupled aromatic compound (3-1) of an embodiment of the present invention comprises:
preparing an aromatic compound (1) having at least two leaving groups;
preparing a compound (2) capable of undergoing a cross-coupling reaction selected from an aromatic boronic acid (2-1), an aromatic amino compound (2-2), a diboronic acid ester (2-3), an aromatic compound (2-4) having a hydroxyl group and an aromatic compound (2-5) having a thiol group; and
performing a cross-coupling reaction of the aromatic compound (1) having at least two leaving groups with the compound (2) in the presence of a palladium catalyst and a base, in the absence of a solvent. The mono-cross-coupled aromatic compound (3-1) has one less leaving group than the aromatic compound (1) having at least two leaving groups (the number of leaving group(s) which the mono-cross-coupled aromatic compound (3-1) has is smaller than that of leaving groups which the aromatic compound (1) has, and the difference between the number of leaving group(s) which the mono-cross-coupled aromatic compound (3-1) has and the number of leaving groups which the aromatic compound (1) has is one).

The method for producing a mono-cross-coupled aromatic compound (3-1) of the embodiment of the present invention comprises:
<1> preparing an aromatic compound (1) having at least two leaving groups.

In the present disclosure, the aromatic compound (1) having at least two leaving groups is not particularly limited as long as it has an aromatic group bonded to at least two leaving groups, and the objective cross-coupling reaction of the present invention can be performed.

In the present disclosure, the leaving group is not particularly limited as long as the objective cross-coupling reaction of the present invention can be performed, and can be generally selected from a halogeno group such as chloro, bromo or iodo, a diazonium salt, trifluoromethanesulfonate and a carboxylic acid derivative. The leaving group can also be selected from a halogeno group such as chloro, bromo or iodo, a diazonium salt and trifluoromethanesulfonate, and the halogeno group is preferable.

In the present disclosure, the halogeno group as the leaving group can be generally selected from chloro, bromo and iodo. Two halogeno groups may be the same or different. Both may be chloro, bromo, iodo, a combination of chloro and bromo, a combination of chloro and iodo, or a combination of bromo and iodo. Both may be the same halogeno groups, for example, both are chloro, bromo or iodo.

In the embodiment of the present invention, there is provided a method for producing a mono-cross-coupled aromatic compound (3-1), wherein the aromatic compound (1) having at least two leaving groups is a halogenated aromatic compound in which the leaving group is halogen, and the mono-cross-coupled aromatic compound (3-1) is a halogenated mono-cross-coupled aromatic compound.

The aromatic group is an aromatic group which is usually used in a cross-coupling reaction, and is not particularly limited as long as the objective cross-coupling reaction of the present invention can be performed. The aromatic group can be generally selected from an optionally substituted aromatic group and an optionally substituted heteroaromatic group (an aromatic group containing a heteroatom).

Examples of the optionally substituted aromatic group include a phenyl group, a naphthalene group, an anthracene group, a phenanthrene group, a biphenyl group, a terphenyl group, a pyrene group, a perylene group, a triphenylene group and the like. Regarding the optionally substituted aromatic group, the number of hydrogens corresponding to the number of substituents are eliminated.

Examples of the optionally substituted heteroaromatic group include:
sulfur-containing heteroaromatic groups such as a thiophene group, a benzothiophene group, a dibenzothiophene group and a phenyldibenzothiophene group;
oxygen-containing heteroaromatic groups such as a furan group, a benzofuran group, a dibenzofuran group and a phenyldibenzofuran group;
nitrogen-containing heteroaromatic groups such as a pyridine group, a pyrimidine group, a pyrazine group, a quinoline group, an isoquinoline group, a carbazole group, a 9-phenylcarbazole group, an acridine group, a quinazoline group, a quinoxaline group, a 1,6-naphthyridine group, a 1,8-naphthyridinegroup and a porphyrin group; and
heteroaromatic groups containing two or more heteroatoms (e.g., nitrogen and sulfur), such as a benzothiazole group. Regarding the optionally substituted heteroaromatic group, the number of hydrogens corresponding to the number of substituents are eliminated.

There is no particular limitation on the substituent, with which the optionally substituted aromatic group and the optionally substituted heteroaromatic group can be substituted, as long as the objective cross-coupling reaction of the present invention can be performed.

Examples of the substituent include:
alkyl groups having 1 to 24 carbon atoms, for example, 1 to 18 carbon atoms, for example, 1 to 12 carbon atoms, for example, 1 to 8 carbon atoms (e.g., a methyl group, an ethyl group, an n-propyl group, an isopropyl group, an n-butyl group, an isobutyl group, a tert-butyl group, a pentyl group, a hexyl group, an octyl group, etc.),
alkoxy groups having 1 to 24 carbon atoms, for example, 1 to 18 carbon atoms, for example, 1 to 12 carbon atoms, for example, 1 to 8 carbon atoms (e.g., a methoxy group, an ethoxy group, an n-propoxy group, an isopropoxy group, an n-butoxy group, an isobutoxy group, a tert-butoxy group, a pentyloxy group, a hexyloxy group, an octyloxy group, etc.),
cycloalkyl groups having 3 to 24 carbon atoms, for example, 3 to 18 carbon atoms, for example, 3 to 12 carbon atoms, for example, 3 to 8 carbon atoms (e.g., a cyclopropyloxy group, a cyclobutyloxy group, a cyclopentyloxy group, a cyclohexyloxy group, etc.),
alkenyl groups having 1 to 24 carbon atoms, for example, 1 to 18 carbon atoms, for example, 1 to 12 carbon atoms, for example, 1 to 8 carbon atoms (e.g., an ethenyl group, a propenyl group, a butenyl group, a pentenyl group, a hexenyl group, an octenyl group, etc.),
alkynyl groups having 1 to 24 carbon atoms, for example, 1 to 18 carbon atoms, for example, 1 to 12 carbon atoms, for example, 1 to 8 carbon atoms (e.g., an ethynyl group, a propynyl group, a butynyl group, a pentynyl group, a hexynyl group, an octynyl group, etc.),
aryl groups having 5 to 24 carbon atoms, for example, 5 to 18 carbon atoms, for example, 5 to 12 carbon atoms, for example, 5 to 8 carbon atoms (e.g., a phenyl group, a naphthyl group, a biphenyl group, etc.),
aryloxy groups having 5 to 24 carbon atoms, for example, 5 to 18 carbon atoms, for example, 5 to 12 carbon atoms, for example, 5 to 8 carbon atoms (e.g., a phenoxy group, a naphthyloxy group, a biphenyloxy group, etc.),
heteroaryl groups having 4 to 24 carbon atoms, for example, 1 to 18 carbon atoms, for example, 1 to 12 carbon atoms, for example, 1 to 8 carbon atoms (e.g., a thiophenyl group, a furanyl group, a carbazole group, a benzothiophenyl group, a benzofuranyl group, an indolyl group, a pyrrolyl group, a pyridyl group, etc.),
acyl groups having 1 to 24 carbon atoms, for example, 1 to 18 carbon atoms, for example, 1 to 12 carbon atoms, for example, 1 to 8 carbon atoms (e.g., an acetyl group, a propionyl group, a butanoyl group, a pentanoyl group, a heptanoyl group, and a group in which a carbonyl group included in the acyl group is substituted with an ester group or an amide group),
amino groups having 1 to 24 carbon atoms, for example, 1 to 18 carbon atoms, for example, 1 to 12 carbon atoms, for example, 1 to 8 carbon atoms (e.g., a diphenylamino group, a dimethylamino group, etc.), and
fluorine (including partial fluorine substitution and complete fluorine substitution), cyano groups and nitro groups.

The substituents may be crosslinked to each other, and the entire substituent may form a cyclic structure (an aromatic group). Further, the above-mentioned substituent may be further substituted with the above-mentioned substituent.

In the embodiment of the present invention, there is provided a method for producing a mono-cross-coupled aromatic compound (3-1), wherein the aromatic compound (1) having at least two leaving groups contains two leaving groups and an aromatic group,
two leaving groups are selected from a halogeno group, a diazonium salt, trifluoromethanesulfonate and a carboxylic acid derivative, and two leaving groups may be the same or different.

Preferred is the case of having two leaving groups, and the aromatic group bonded to two leaving groups is not particularly limited as long as it is a disubstituted aromatic group which is usually used in the cross-coupling reaction and is a group having a cyclic structure with aromaticity, and the objective cross-coupling reaction of the present invention can be performed. The disubstituted aromatic group can be generally selected from an optionally substituted aromatic group containing no heteroatom (an arenediyl group or an arylene group) and an optionally substituted aromatic group containing a heteroatom (or a heteroaromatic group).

Examples of the optionally disubstituted aromatic group containing no heteroatom include a phenylene group, a naphthalenediyl group, an anthracenediyl group, a phenanthrenediyl group, a biphenyldiyl group, a terphenyldiyl group, a pyrenediyl group, a perylenediyl group, a triphenylenediyl group and the like.

Examples of the optionally disubstituted heteroaromatic group include:
sulfur-containing heteroaromatic groups such as a thiophenediyl group, a benzothiophenediyl group, a dibenzothiophenediyl group and a phenyldibenzothiophenediyl group;
oxygen-containing heteroaromatic groups such as a furandiyl group, a benzofurandiyl group, a dibenzofurandiyl group and a phenyldibenzofurandiyl group;
nitrogen-containing heteroaromatic groups such as a pyridinediyl group, a pyrimidinediyl group, a pyrazinediyl group, a quinolinediyl group, an isoquinolinediyl group, a carbazolediyl group, a 9-phenylcarbazolediyl group, an acridinediyl group, a quinazolinediyl group, a quinoxalinediyl group, a 1,6-naphthyridinediyl group, a 1,8-naphthyridinediyl group and a porphyrindiyl group (or a porphyrin ring); and
heteroaromatic groups containing two or more heteroatoms (e.g., nitrogen and sulfur), such as a benzothiazolediyl group.

There is no particular limitation on the substituent, with which the disubstituted aromatic group containing no heteroatom and the heteroaromatic group can be substituted, as long as the objective cross-coupling reaction of the present invention can be performed.

It is possible to exemplify, as the substituent, for example, substituents, with which the above-mentioned optionally substituted aromatic group and optionally substituted heteroaromatic group can be substituted.

The aromatic compound (1) having at least two leaving groups can be more specifically represented, for example, by the following general formula (I):

A¹ -Xn : (I)

wherein A¹ is selected from an optionally substituted aromatic group and an optionally substituted heteroaromatic group, X is selected from a halogeno group, a diazonium salt, trifluoromethanesulfonate and a carboxylic acid derivative, n is an integer of 2 or more, for example, 4 or less, 3 or less or may be 2.

In formula (I), the optionally substituted aromatic group and the optionally substituted heteroaromatic group as for A¹ can include, for example, the above-mentioned optionally substituted aromatic group and optionally substituted heteroaromatic group.

In formula (I), the substituent of the optionally substituted aromatic group and optionally substituted heteroaromatic group can be, for example, the above-mentioned substituents.

The substituents may be crosslinked to each other, and the entire substituent may form a cyclic structure (an aromatic group). Further, the above-mentioned substituent may be further substituted with the above-mentioned substituent.

In formula (I), X is preferably selected from a halogeno group.

Preferred is the case of having two leaving groups, and it is possible to further exemplify, as A¹ of the aromatic compound (1) having at least two leaving groups, for example, the following disubstituted groups:
naphthalenediyl groups such as a naphthalenediyl group, an aryl (e.g., phenyl, etc.) naphthalenediyl group, a naphthalenediyl group having an alkylene (e.g., ethylene, etc.) crosslink, and a naphthalenediyl group having an arylene (e.g., phenylene, etc.) crosslink;
phenanthrenediyl groups;
anthracenediyl groups such as an anthracenediyl group, an aryl (e.g., phenyl, etc.) anthracenediyl group, a diaryl (e.g., dinaphthyl, etc.) anthracenediyl group, and a diarylboryl (e.g., bis(trialkylphenyl)boryl, etc.) anthracenediyl group;
pyrenediyl groups such as a pyrenediyl group and an alkyl (e.g., t-butyl, etc.) pyrenediyl group;
biphenyldiyl groups such as a biphenyldiyl group and a biphenyldiyl group having an alkylene (e.g., propyrene, isopropyrene, etc.) crosslink;
terphenyldiyl groups such as a terphenyldiyl group and a tetraaryl (e.g., tetraphenyl, etc.) terphenyldiyl group;
triphenylenediyl groups;
phenylene groups such as a 2-aryl (e.g., phenyl, etc.) ethenylphenylene group, a 1,2,2-triaryl (e.g., triphenyl, etc.) ethenylphenylene group, a 2-aryl (e.g., phenyl, etc.) ethynylphenylene group, a phenylene group, an alkyl (e.g., methyl) phenylene group, a dialkyl (e.g., dimethyl) phenylene group, an alkoxy (e.g., methoxy) phenylene group, a dialkylamino (e.g., dimethylamino) phenylene group, a diaryl (e.g., diphenyl) aminophenylene group, a perfluoroalkyl (e.g., trifluoromethyl) phenylene group, an alkyl (e.g., ethyl) oxycarbonylphenylene group, and an alkanoyl (e.g., acyl) phenylene group;
carbazolediyl groups and aryl (e.g., phenyl, etc.)-substituted carbazolediyl groups;
anthracenediyl groups and anthracene-9.10-dionediyl groups,
thiophenediyl groups and aryl (e.g., phenyl, etc.)-substituted thiophenediyl groups; and
porphyrindiyl groups and aryl (e.g., bis(3,5-methylphenyl), etc.) porphyrindiyl groups.

It is possible to use, as the aromatic compound (1) having at least two leaving groups, commercially available compounds.

The method for producing a monohalogenated aromatic compound (3-1) of the embodiment of the present invention comprises:
<2> preparing a compound (2) capable of undergoing a cross-coupling reaction selected from an aromatic boronic acids (including an acid and an ester thereof) (2-1), an aromatic amino compound (2-2), a diboronic acid ester (including an ester and an acid) (2-3), an aromatic compound (2-4) having a hydroxyl group, and an aromatic compound (2-5) having a thiol group.

In the present disclosure, the compound (2) capable of undergoing a cross-coupling reaction is not particularly limited as long as it is a compound capable of undergoing a cross-coupling reaction with the above-mentioned aromatic compound (1) having at least two leaving groups, and corresponds to compounds (2-1) to (2-5) selected from an aromatic boronic acid (including an acid and an ester thereof) (2-1), an aromatic amino compound (2-2), a diboronic acid ester (including an ester and an acid) (2-3), an aromatic compound (2-4) having a hydroxyl group and an aromatic compound (2-5) having a thiol group.

In the present disclosure, the aromatic boronic acid (including an acid or an ester thereof) (2-1) is not particularly limited as long as it has a B-C bond and has an aromatic group, and undergoes a cross-coupling reaction with the above-mentioned dihalogenated aromatic compound (1) to give a cross-coupled product in which a C-C bond is formed. In the present disclosure, the aromatic boronic acid (2-1) includes both an aromatic boronic acid and an aromatic boronic acid ester. Examples of the aromatic boronic acid ester include an aromatic boronic acid alkyl ester, an aromatic boronic acid alkylene glycol ester, an aromatic boronic acid aryl ester and an aromatic boronic acid arylene glycol ester.

The aromatic group can be selected, for example, from an optionally substituted aryl group (or an aromatic group) and an optionally substituted heteroaryl group (or a heteroaromatic group).

Examples of the optionally substituted aryl group (or the aromatic hydrocarbon group) include a phenyl group, a naphthyl group, an anthracenyl group (or an anthracene group), a phenanthrenyl group (or a phenanthrene group), a biphenyl group, a terphenyl group, a pyrenyl group (or a pyrene group), a perylenyl group (or a perylene group), a triphenylenyl group (or a triphenylene group) and the like.

Examples of the optionally substituted heteroaryl group (or the heteroaromatic group) include:
sulfur-containing heteroaryl groups such as a thiophenyl group (a thiophene group or a thienyl group), a benzothienyl group, a dibenzothienyl group, a phenyldibenzothienylenyl group and a dibenzothienylenylphenyl group;
oxygen-containing heteroaryl groups such as a furanyl group, a benzofuranyl group, a dibenzofuranyl group, a phenyldibenzofuranyl group and a dibenzofuranylphenyl group;
nitrogen-containing heteroaryl groups such as a pyridyl group (or a pyridine group), a pyridylenyl group (or a pyridinediyl group), a pyrimidinyl group (or a pyrimidine group), a pyrazyl group (or a pyrazine group), a quinolyl group (or a quinoline group), an isoquinolyl group (or an isoquinoline group), a carbazolyl group (or a carbazole group), a 9-phenylcarbazolyl group, an acridinyl group (or an acrydine group), a quinazolyl group (or a quinazoline group), a quinoxalyl group (or a quinoxaline group), a 1,6-naphthyridinyl group, a 1,8-naphthyridinyl group and a porphyrin group (or a porphyrin ring); and
heteroaryl groups containing two or more heteroatoms (e.g., nitrogen and sulfur), such as a benzothiazolyl group (or a benzothiazole group).

There is no particular limitation on the substituent, with which the aryl group and the heteroaryl group can be substituted, as long as the objective cross-coupling reaction of the present invention can be performed.

Examples of the substituent include:
alkyl groups having 1 to 24 carbon atoms, for example, 1 to 18 carbon atoms, for example, 1 to 12 carbon atoms, for example, 1 to 8 carbon atoms (e.g., a methyl group, an ethyl group, an n-propyl group, an isopropyl group, an n-butyl group, an isobutyl group, a tert-butyl group, a pentyl group, a hexyl group, an octyl group, etc.),
alkoxy groups having 1 to 24 carbon atoms, for example, 1 to 18 carbon atoms, for example, 1 to 12 carbon atoms, for example, 1 to 8 carbon atoms (e.g., a methoxy group, an ethoxy group, an n-propoxy group, an isopropoxy group, an n-butoxy group, an isobutoxy group, a tert-butoxy group, a pentyloxy group, a hexyloxy group, an octyloxy group, etc.),
cycloalkyl groups having 3 to 24 carbon atoms, for example, 3 to 18 carbon atoms, for example, 3 to 12 carbon atoms, for example, 3 to 8 carbon atoms (e.g., a cyclopropyloxy group, a cyclobutyloxy group, a cyclopentyloxy group, a cyclohexyloxy group, etc.),
alkenyl groups having 1 to 24 carbon atoms, for example, 1 to 18 carbon atoms, for example, 1 to 12 carbon atoms, for example, 1 to 8 carbon atoms (e.g., an ethenyl group, a propenyl group, a butenyl group, a pentenyl group, a hexenyl group, an octenyl group, etc.),
alkynyl groups having 1 to 24 carbon atoms, for example, 1 to 18 carbon atoms, for example, 1 to 12 carbon atoms, for example, 1 to 8 carbon atoms (e.g., an ethynyl group, a propynyl group, a butynyl group, a pentynyl group, a hexynyl group, an octynyl group, etc.),
aryl groups having 5 to 24 carbon atoms, for example, 5 to 18 carbon atoms, for example, 5 to 12 carbon atoms, for example, 5 to 8 carbon atoms (e.g., a phenyl group, a naphthyl group, a biphenyl group, etc.),
aryloxy groups having 5 to 24 carbon atoms, for example, 5 to 18 carbon atoms, for example, 5 to 12 carbon atoms, for example, 5 to 8 carbon atoms (e.g., a phenoxy group, a naphthyloxy group, a biphenyloxy group, etc.),
heteroaryl groups having 4 to 24 carbon atoms, for example, 1 to 18 carbon atoms, for example, 1 to 12 carbon atoms, for example, 1 to 8 carbon atoms (e.g., a thiophenyl group, a furanyl group, a carbazole group, a benzothiophenyl group, a benzofuranyl group, an indolyl group, a pyrrolyl group, a pyridyl group, etc.),
acyl groups having 1 to 24 carbon atoms, for example, 1 to 18 carbon atoms, for example, 1 to 12 carbon atoms, for example, 1 to 8 carbon atoms (e.g., an acetyl group, a propionyl group, a butanoyl group, a pentanoyl group, a heptanoyl group, a group in which a carbonyl group included in the acyl group is substituted with an ester group or an amide group, etc.),
amino groups having 1 to 24 carbon atoms, for example, 1 to 18 carbon atoms, for example, 1 to 12 carbon atoms, for example, 1 to 8 carbon atoms (e.g., a diphenylamino group, a dimethylamino group, etc.), and
fluorine (including partial fluorine substitution and complete fluorine substitution), cyano groups and nitro groups.

In the embodiment of the present invention, the aromatic boronic acid (including an acid or an ester thereof) (2-1) can be more specifically represented, for example, by the general formula (II-1): 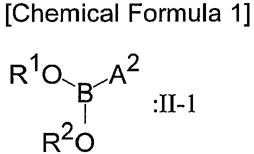 wherein R¹ to R² are each independently selected from hydrogen, an optionally substituted alkyl group and an optionally substituted aryl group, and R¹ and R² may be bonded to each other, and

A² is selected from an optionally substituted aryl group and an optionally substituted heteroaryl group.

In formula (II-1), R¹ to R² are each independently selected from hydrogen, an optionally substituted alkyl group and an optionally substituted aryl group (or an aromatic hydrocarbon group), R¹ and R² may be bonded to each other. Further, R¹ and R² may combine to form a cyclic structure. The cyclic structure may be an aromatic group. For example, a 1,2-phenylene group and the like can be exemplified.

Examples of the optionally substituted alkyl group include a methyl group, an ethyl group, a propyl group, an isopropyl group, a butyl group, an isobutyl group, a t-butyl group and the like. Furthermore, two alkyl groups may be bonded, and examples of R¹-R² include an ethylene group, a 1,1,2,2-tetramethylethylene group (a pinacolato group), a neopentyl glycolate group and a propyrene group.

The optionally substituted aryl group is selected, for example, from a phenyl group, a naphthyl group, a biphenyl group and the like.

Both the alkyl group and the aryl group are optionally substituted. The substituent can be selected from an alkyl group, an aryl group, an alkoxy group, an aryloxy group and the like. The substituents may be crosslinked to each other. The substituent can be further substituted.

In formula (II-1), A² is not particularly limited as long as it is selected from an optionally substituted aryl group and an optionally substituted heteroaryl group, and a cross-coupling reaction can be performed.

The optionally substituted aryl group and the optionally substituted heteroaryl group can include the above-mentioned optionally substituted aryl group and optionally substituted heteroaryl group.

In formula (II-1), the substituent of the optionally substituted aryl group and the optionally substituted heteroaryl group can be, for example, the above-mentioned substituent.

Further, the substituent may be further substituted with the above-mentioned substituent.

It is possible to more specifically exemplify, as A² of the aromatic boronic acid (II-1), for example, the following groups:
naphthyl groups such as a naphthyl group, an aryl (e.g., phenyl, etc.) naphthyl group, a naphthyl group having an alkylene (e.g., ethylene, etc.) crosslink, and a naphthyl group having an arylene (e.g., phenylene, etc.) crosslink;
phenanthrenyl groups;
anthracenyl groups such as an anthracenyl group, an aryl (e.g., phenyl, etc.) anthracenyl group, a diaryl (e.g., dinaphthyl, etc.) anthracenyl group, and a diarylboryl (e.g., bis(trialkylphenyl)boryl, etc.) anthracenyl group;
pyrenyl groups such as a pyrenyl group and an alkyl (e.g., t-butyl) pyrenyl group;
biphenyl groups such as a biphenyl group, and a biphenyl group having an alkylene (e.g., propyrene, isopropyrene, etc.) crosslink;
terphenyl groups such as a terphenyl group and a tetraaryl (e.g., tetraphenyl, etc.) terphenyl group;
triphenylenyl groups;
phenyl groups such as a 2-aryl (e.g., phenyl, etc.) ethenylphenyl group, a 1,2,2-triaryl (e.g., triphenyl, etc.) ethenylphenyl group, a 2-aryl (e.g., phenyl, etc.) ethynylphenyl group, a phenyl group, an alkyl (e.g., methyl) phenyl group, a dialkyl (e.g., dimethyl) phenyl group, an alkoxy (e.g., methoxy) phenyl group, a dialkylamino (e.g., dimethylamino) phenyl group, a diaryl (e.g., diphenyl) aminophenyl group, a perfluoroalkyl (e.g., trifluoromethyl) phenyl group, an alkyl (e.g., ethyl) oxycarbonylphenyl group, and an alkanoyl (e.g., acyl) phenyl group;
aryl (e.g., phenyl, etc.)-substituted carbazolyl groups;
anthracene-9.10-dione groups;
thienyl groups, aryl (e.g., phenyl, etc.)-substituted thienyl groups and the like.

It is possible to use, as the aromatic boronic acid (2-1), commercially available compounds.

In the present disclosure, the aromatic amino compound (2-2) is not particularly limited as long as it has an amino group bonded to an aromatic group, the amino group has hydrogen, and undergoes a cross-coupling reaction with the above-mentioned dihalogenated aromatic compound (1) to give a cross-coupled product in which an N-C bond is formed.

The aromatic group can be selected, for example, from an optionally substituted aryl group (or an aromatic hydrocarbon group) and an optionally substituted heteroaryl group (or a heteroaromatic group). The aromatic amino compound (2-2) can have up to two aromatic groups. When the aromatic amino compound (2-2) has two aromatic groups, two aromatic groups may be either crosslinked or bonded to each other.

The aromatic amino compound (2-2) has two substituents, which may be bonded to each other to entirely form one cyclic structure, for example, an aromatic group.

Examples of the optionally substituted aryl group (or the aromatic hydrocarbon group) include a phenyl group, a naphthyl group, an anthracenyl group (or an anthracene group), a phenanthrenyl group (or a phenanthrene group), a biphenyl group, a terphenyl group, a pyrenyl group (or a pyrene group), a perylenyl group (or a perylene group), a triphenylenyl group (or a triphenylene group) and the like.

Examples of the optionally substituted heteroaryl group (or the heteroaromatic group) include:
sulfur-containing heteroaryl groups such as a thiophenyl group (a thiophene group or a thienyl group), a benzothienyl group, a dibenzothienyl group, a phenyldibenzothienylenyl group and a dibenzothienylenylphenyl group;
oxygen-containing heteroaryl groups such as a furanyl group (or a furan group), a benzofuranyl group, a dibenzofuranyl group, a phenyldibenzofuranyl group and a dibenzofuranylphenyl group;
nitrogen-containing heteroaryl groups such as a pyridyl group (or a pyridine group), a pyridylenyl group (or a pyridinediyl group), a pyrimidinyl group (or a pyrimidine group), a pyrazyl group (or a pyrazine group), a quinolyl group (or a quinoline group), an isoquinolyl group (or an isoquinoline group), a carbazolyl group (or a carbazole group), a 9-phenylcarbazolyl group, an acridinyl group (or an acrydine group), a quinazolyl group (or a quinazoline group), a quinoxalyl group (or a quinoxaline group), a 1,6-naphthyridinyl group, a 1,8-naphthyridinyl group and a porphyrin group (or a porphyrin ring); and
heteroaryl groups containing two or more heteroatoms (e.g., nitrogen and sulfur), such as a benzothiazolyl group (or a benzothiazole group).

There is no particular limitation on the substituent, with which the aryl group and the heteroaryl group can be substituted, as long as the objective cross-coupling reaction of the present invention can be performed.

Examples of the substituent include:
alkyl groups having 1 to 24 carbon atoms, for example, 1 to 18 carbon atoms, for example, 1 to 12 carbon atoms, for example, 1 to 8 carbon atoms (e.g., a methyl group, an ethyl group, an n-propyl group, an isopropyl group, an n-butyl group, an isobutyl group, a tert-butyl group, a pentyl group, a hexyl group, an octyl group, etc.),
alkoxy groups having 1 to 24 carbon atoms, for example, 1 to 18 carbon atoms, for example, 1 to 12 carbon atoms, for example, 1 to 8 carbon atoms (e.g., a methoxy group, an ethoxy group, an n-propoxy group, an isopropoxy group, an n-butoxy group, an isobutoxy group, a tert-butoxy group, a pentyloxy group, a hexyloxy group, an octyloxy group, etc.),
cycloalkyl groups having 3 to 24 carbon atoms, for example, 3 to 18 carbon atoms, for example, 3 to 12 carbon atoms, for example, 3 to 8 carbon atoms (e.g., a cyclopropyloxy group, a cyclobutyloxy group, a cyclopentyloxy group, a cyclohexyloxy group, etc.),
alkenyl groups having 1 to 24 carbon atoms, for example, 1 to 18 carbon atoms, for example, 1 to 12 carbon atoms, for example, 1 to 8 carbon atoms (e.g., an ethenyl group, a propenyl group, a butenyl group, a pentenyl group, a hexenyl group, an octenyl group, etc.),
alkynyl groups having 1 to 24 carbon atoms, for example, 1 to 18 carbon atoms, for example, 1 to 12 carbon atoms, for example, 1 to 8 carbon atoms (e.g., an ethynyl group, a propynyl group, a butynyl group, a pentynyl group, a hexynyl group, an octynyl group, etc.),
aryl groups having 5 to 24 carbon atoms, for example, 5 to 18 carbon atoms, for example, 5 to 12 carbon atoms, for example, 5 to 8 carbon atoms (e.g., a phenyl group, a naphthyl group, a biphenyl group, etc.),
aryloxy groups having 5 to 24 carbon atoms, for example, 5 to 18 carbon atoms, for example, 5 to 12 carbon atoms, for example, 5 to 8 carbon atoms (e.g., a phenoxy group, a naphthyloxy group, a biphenyloxy group, etc.),
heteroaryl groups having 4 to 24 carbon atoms, for example, 1 to 18 carbon atoms, for example, 1 to 12 carbon atoms, for example, 1 to 8 carbon atoms (e.g., a thiophenyl group, a furanyl group, a carbazole group, a benzothiophenyl group, a benzofuranyl group, an indolyl group, a pyrrolyl group, a pyridyl group, etc.),
acyl groups having 1 to 24 carbon atoms, for example, 1 to 18 carbon atoms, for example, 1 to 12 carbon atoms, for example, 1 to 8 carbon atoms (e.g., an acetyl group, a propionyl group, a butanoyl group, a pentanoyl group, a heptanoyl group, a group in which a carbonyl group included in the acyl group is substituted with an ester group or an amide group, etc.),
amino groups having 1 to 24 carbon atoms, for example, 1 to 18 carbon atoms, for example, 1 to 12 carbon atoms, for example, 1 to 8 carbon atoms (e.g., a diphenylamino group, a dimethylamino group, etc.), and
fluorine (including partial fluorine substitution and complete fluorine substitution), cyano groups and nitro groups.

The substituents may be crosslinked to each other, and the entire substituent may form a cyclic structure (an aromatic group). Further, the above-mentioned substituent may be further substituted with the above-mentioned substituent.

The aromatic amino compound (2-2) is more specifically represented, for example, by the general formula (II-2): 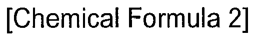 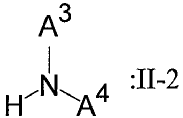 wherein A³ and A⁴ are each independently selected from hydrogen, an optionally substituted aryl group (or an aromatic hydrocarbon group) and an optionally substituted heteroaryl group (or a heteroaromatic group), A³ and A⁴ are not simultaneously hydrogen, and A³ and A⁴ may be bonded to each other.

In formula (II-1), A³ and A⁴ are selected from hydrogen, an optionally substituted aryl group and an optionally substituted heteroaryl group, but are not simultaneously hydrogen.

The optionally substituted aryl group and the optionally substituted heteroaryl group can include the above-mentioned optionally substituted aryl group and optionally substituted heteroaryl group.

In formula (II-2), the substituent in the optionally substituted aryl group and the optionally substituted heteroaryl group can be, for example, the above-mentioned substituent.

The substituents may be crosslinked to each other, and the entire substituent may form a cyclic structure (an aromatic group). Further, the above-mentioned substituent may be further substituted with the above-mentioned substituent.

It is possible to more specifically exemplify, as A³ and A⁴ of the aromatic amino compound (2-2), for example, the following groups:
naphthyl groups such as a naphthyl group, an aryl (e.g., phenyl, etc.) naphthyl group, a naphthyl group having an alkylene (e.g., ethylene, etc.) crosslink, and a naphthyl group having an arylene (e.g., phenylene, etc.) crosslink;
phenanthrenyl groups;
anthracenyl groups such as an anthracenyl group, an aryl (e.g., phenyl, etc.) anthracenyl group, a diaryl (e.g., dinaphthyl, etc.) anthracenyl group, and a diarylboryl (e.g., bis(trialkylphenyl)boryl, etc.) anthracenyl group;
pyrenyl groups such as a pyrenyl group and an alkyl (e.g., t-butyl, etc.) pyrenyl group;
biphenyl groups such as a biphenyl group, and a biphenyl group having an alkylene (e.g., propyrene, isopropyrene, etc.) crosslink;
terphenyl groups such as a terphenyl group and a tetraaryl (e.g., tetraphenyl, etc.) terphenyl group;
triphenylenyl groups;
phenyl groups such as a 2-aryl (e.g., phenyl, etc.) ethenylphenyl group, a 1,2,2-triaryl (e.g., triphenyl, etc.) ethenylphenyl group, a 2-aryl (e.g., phenyl, etc.) ethynylphenyl group, a phenyl group, an alkyl (e.g., methyl) phenyl group, a dialkyl (e.g., dimethyl) phenyl group, an alkoxy (e.g., methoxy) phenyl group, a dialkylamino (e.g., dimethylamino) phenyl group, a diaryl (e.g., diphenyl) aminophenyl group, a perfluoroalkyl (e.g., trifluoromethyl) phenyl group, an alkyl (e.g., ethyl) oxycarbonylphenyl group, and an alkanoyl (e.g., acyl) phenyl group;
aryl (e.g., phenyl, etc.)-substituted carbazolyl groups;
anthracene-9.10-dione groups, and
aryl (e.g., phenyl, etc.)-substituted thienyl groups.

It is possible to use, as the aromatic amino compound (2-1), commercially available compounds.

In the present disclosure, the diboronic acid ester (2-3) is not particularly limited as long as it has a B-B bond and undergoes a cross-coupling reaction with the above-mentioned dihalogenated aromatic compound (1) to give a cross-coupled product in which a B-C bond is formed.

In the present disclosure, the diboronic acid ester (2-3) includes diboronic acid monoester and diboronic acid and includes, for example, tetrahydroxydiborane.

Examples of the diboronic acid ester (2-3) include a diboronic acid alkyl ester, a diboronic acid alkylene glycol ester, a diboronic acid aryl ester, diboronic acid arylene glycol ester and tetrahydroxydiborane.

In the embodiment of the present invention, the diboronic acid esters (including an ester and an acid) (2-3) can be more specifically represented, for example, by the general formula (II-3): 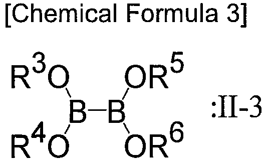 wherein R³ to R⁶ are each independently selected from hydrogen, an optionally substituted alkyl group and an optionally substituted aryl group (or an aromatic hydrocarbon group), R³ and R⁴ may be bonded to each other, and R⁵ and R⁶ may be bonded to each other.

In formula (II-3), R³ to R⁶ are each independently selected from hydrogen, an optionally substituted alkyl group and an optionally substituted aryl group, R³ and R⁴ may be bonded to each other, and R⁵ and R⁶ may be bonded to each other. Further, R³ and R⁴ may combine together to form a cyclic structure, and R⁵ and R⁶ may combine together to form a cyclic structure. The cyclic structure may be an aromatic group. For example, a 1,2-phenylene group and the like can be exemplified.

Examples of the optionally substituted alkyl group include a methyl group, an ethyl group, a propyl group, an isopropyl group, a butyl group, an isobutyl group, a t-butyl group and the like. Further, two alkyl groups may be bonded, and examples of R¹ -R² and R³-R⁴ include an ethylene group, a 1,1,2,2-tetramethylethylene group, a 2,2-dimethylpropyrene group, a hexylene group (or a 1,1,3-trimethylpropyrene group) and the like.

The optionally substituted aryl group is selected, for example, from a phenyl group, a naphthyl group, a biphenyl group and the like.

The substituent, with which the alkyl group and the aryl group may be substituted, can be selected from an alkyl group, an aryl group, an alkoxy group, an aryloxy group and the like. The substituents may be crosslinked to each other. The above-mentioned substituent may be further substituted with the above-mentioned substituent.

More specifically, examples of the diboronic acid ester includes bis(pinacolato)diboron), bis(neopentyl glycolate)diboron), bis(hexylene glycolato)diboron), bis(catecholato)diboron and the like.

It is possible to use, as the diboronic acid ester (2-3), commercially available products.

In the present disclosure, the aromatic compound (2-4) having a hydroxyl group is not particularly limited as long as it has an H-O bond and has an aromatic group, and undergoes a cross-coupling reaction with the above-mentioned dihalogenated aromatic compound (1) to give a cross-coupled reaction product in which an O-C bond is formed.

The aromatic group can be selected, for example, from an optionally substituted aryl group (or an aromatic hydrocarbon group) and an optionally substituted heteroaryl group (or a heteroaromatic group).

Examples of the optionally substituted aryl group (or the aromatic hydrocarbon group) include a phenyl group, a naphthyl group, an anthracenyl group (or an anthracene group), a phenanthrenyl group (or a phenanthrene group), a biphenyl group, a terphenyl group, a pyrenyl group (or a pyrene group), a perylenyl group (or a perylene group), a triphenylenyl group (or a triphenylene group) and the like.

Examples of the optionally substituted heteroaryl group (or the heteroaromatic group) include:
sulfur-containing heteroaryl groups such as a thiophenyl group (a thiophene group or a thienyl group), a benzothienyl group, a dibenzothienyl group, a phenyldibenzothienylenyl group and a dibenzothienylenylphenyl group;
oxygen-containing heteroaryl groups such as a furanyl group (or a furan group), a benzofuranyl group, a dibenzofuranyl group, a phenyldibenzofuranyl group and a dibenzofuranylphenyl group;
nitrogen-containing heteroaryl groups such as a pyridyl group (or a pyridine group), a pyridylenyl group (or a pyridinediyl group), a pyrimidinyl group (or a pyrimidine group), a pyrazyl group (or a pyrazine group), a quinolyl group (or a quinoline group), an isoquinolyl group (or an isoquinoline group), a carbazolyl group (or a carbazole group), a 9-phenylcarbazolyl group, an acridinyl group (or an acrydine group), a quinazolyl group (or a quinazoline group), a quinoxalyl group (or a quinoxaline group), a 1,6-naphthyridinyl group, a 1,8-naphthyridinyl group and a porphyrin group (or a porphyrin ring); and
heteroaryl groups containing two or more heteroatom (e.g., nitrogen and sulfur), such as a benzothiazolyl group (or a benzothiazole group).

There is no particular limitation on the substituent, with which the aryl group and the heteroaryl group can be substituted, as long as the objective cross-coupling reaction of the present invention can be performed.

Examples of the substituent include:
alkyl groups having 1 to 24 carbon atoms, for example, 1 to 18 carbon atoms, for example, 1 to 12 carbon atoms, for example, 1 to 8 carbon atoms (e.g., a methyl group, an ethyl group, an n-propyl group, an isopropyl group, an n-butyl group, an isobutyl group, a tert-butyl group, a pentyl group, a hexyl group, an octyl group, etc.),
alkoxy groups having 1 to 24 carbon atoms, for example, 1 to 18 carbon atoms, for example, 1 to 12 carbon atoms, for example, 1 to 8 carbon atoms (e.g., a methoxy group, an ethoxy group, an n-propoxy group, an isopropoxy group, an n-butoxy group, an isobutoxy group, a tert-butoxy group, a pentyloxy group, a hexyloxy group, an octyloxy group, etc.),
cycloalkyl groups having 3 to 24 carbon atoms, for example, 3 to 18 carbon atoms, for example, 3 to 12 carbon atoms, for example, 3 to 8 carbon atoms (e.g., a cyclopropyloxy group, a cyclobutyloxy group, a cyclopentyloxy group, a cyclohexyloxy group, etc.),
alkenyl groups having 1 to 24 carbon atoms, for example, 1 to 18 carbon atoms, for example, 1 to 12 carbon atoms, for example, 1 to 8 carbon atoms (e.g., an ethenyl group, a propenyl group, a butenyl group, a pentenyl group, a hexenyl group, an octenyl group, etc.),
alkynyl groups 1 to 24 carbon atoms, for example, 1 to 18 carbon atoms, for example, 1 to 12 carbon atoms, for example, 1 to 8 carbon atoms (e.g., an ethynyl group, a propynyl group, a butynyl group, a pentynyl group, a hexynyl group, an octynyl group, etc.),
aryl groups having 5 to 24 carbon atoms, for example, 5 to 18 carbon atoms, for example, 5 to 12 carbon atoms, for example, 5 to 8 carbon atoms (e.g., a phenyl group, a naphthyl group, a biphenyl group, etc.),
aryloxy groups having 5 to 24 carbon atoms, for example, 5 to 18 carbon atoms, for example, 5 to 12 carbon atoms, for example, 5 to 8 carbon atoms (e.g., a phenoxy group, a naphthyloxy group, a biphenyloxy group, etc.),

heteroaryl groups having 4 to 24 carbon atoms, for example, 1 to 18 carbon atoms, for example, 1 to 12 carbon atoms, for example, 1 to 8 carbon atoms (e.g., a thiophenyl group, a furanyl group, a carbazole group, a benzothiophenyl group, a benzofuranyl group, an indolyl group, a pyrrolyl group, a pyridyl group, etc.),
acyl groups having 1 to 24 carbon atoms, for example, 1 to 18 carbon atoms, for example, 1 to 12 carbon atoms, for example, 1 to 8 carbon atoms (e.g., an acetyl group, a propionyl group, a butanoyl group, a pentanoyl group, a heptanoyl group, a group in which a carbonyl group included in the acyl group is substituted with an ester group or an amide group, etc.),
amino groups having 1 to 24 carbon atoms, for example, 1 to 18 carbon atoms, for example, 1 to 12 carbon atoms, for example, 1 to 8 carbon atoms (e.g., a diphenylamino group, a dimethylamino group, etc.), and
fluorine (including partial fluorine substitution and complete fluorine substitution), cyano groups and nitro groups.

In the embodiment of the present invention, the aromatic compound (2-4) having a hydroxyl group can be more specifically represented, for example, by the general formula (II-4):

HO-(Cₘ H_{2 m} )-A⁵

wherein A⁵ is selected from an optionally substituted aryl group and an optionally substituted heteroaryl group, and m is an integer of 0 to 20.

In formula (II-4), m is an integer of 0 to 20, m is preferably 10 or less, and more preferably 4 or less. A⁵ is not particularly limited as long as it can be selected from an optionally substituted aryl group and an optionally substituted heteroaryl group, and a cross-coupling reaction can be performed.

The optionally substituted aryl group and the optionally substituted heteroaryl group can include the above-mentioned optionally substituted aryl group and optionally substituted heteroaryl group.

In formula (II-4), the substituent of the optionally substituted aryl group and the optionally substituted heteroaryl group can be, for example, the above-mentioned substituent.

Further, the substituent may be further substituted with the above-mentioned substituent.

It is possible to more specifically exemplify, as A⁵ of the aromatic compound (2-4) having a hydroxyl group, for example, the following groups:
naphthyl groups such as a naphthyl group, an aryl (e.g., phenyl) naphthyl group, a naphthyl group having an alkylene (e.g., ethylene) crosslink, and a naphthyl group having an arylene (e.g., phenylene) crosslink;
phenanthrenyl groups;
anthracenyl groups such as an anthracenyl group, an aryl (e.g., phenyl)anthracenyl group, a diaryl (e.g., dinaphthyl) anthracenyl group, and a diarylboryl (e.g., bis(trialkylphenyl)boryl) anthracenyl group;
pyrenyl groups such as a pyrenyl group, an alkyl (e.g., t-butyl) pyrenyl group;
biphenyl groups such as a biphenyl group, and a biphenyl group having an alkylene (e.g., propyrene, isopropyrene) crosslink;
terphenyl groups such as a terphenyl group, and a tetraaryl (e.g., tetraphenyl) terphenyl group;
triphenylenyl groups;
phenyl groups such as a 2-aryl (e.g., phenyl) ethenylphenyl group, a 1,2,2-triaryl (e.g., triphenyl) ethenylphenyl group, a 2-aryl (e.g., phenyl) ethynylphenyl group, a phenyl group, an alkyl (e.g., methyl) phenyl group, a dialkyl (e.g., dimethyl) phenyl group, an alkoxy (e.g., methoxy) phenyl group, a dialkylamino (e.g., dimethylamino) phenyl group, a diaryl (e.g., diphenyl) aminophenyl group, a perfluoroalkyl (e.g., trifluoromethyl) phenyl group, an alkyl (e.g., ethyl) oxycarbonylphenyl group, and an alkanoyl (e.g., acyl) phenyl group;
aryl (e.g., phenyl)carbazolyl groups;
anthracene-9.10-dione groups, and
thienyl groups and aryl (e.g., phenyl) thienyl groups.

It is possible to use, as the aromatic compound (2-4) having a hydroxyl group, commercially available compounds.

In the present disclosure, the aromatic compound (2-5) having a thiol group is not particularly limited as long as it has an H-S bond and has an aromatic group, and undergoes a cross-coupling reaction with the above-mentioned dihalogenated aromatic compound (1) to give a cross-coupled reaction product in which an S-C bond is formed.

The aromatic group can be selected, for example, from an optionally substituted aryl group (or aromatic hydrocarbon group) and an optionally substituted heteroaryl group (or a heteroaromatic group).

Examples of the optionally substituted aryl group (or the aromatic hydrocarbon group) include a phenyl group, a naphthyl group, an anthracenyl group (or an anthracene group), a phenanthrenyl group (or a phenanthrene group), a biphenyl group, a terphenyl group, a pyrenyl group (or a pyrene group), a perylenyl group (or a perylene group), a triphenylenyl group (or a triphenylene group) and the like.

Examples of the optionally substituted heteroaryl group (or the heteroaromatic group) include:
sulfur-containing heteroaryl groups such as a thiophenyl group (a thiophene group or a thienyl group), a benzothienyl group, a dibenzothienyl group, a phenyldibenzothienylenyl group and a dibenzothienylenylphenyl group;
oxygen-containing heteroaryl groups such as a furanyl group (or a furan group), a benzofuranyl group, a dibenzofuranyl group, a phenyldibenzofuranyl group and a dibenzofuranylphenyl group;
nitrogen-containing heteroaryl groups such as a pyridyl group (or a pyridine group), a pyridylenyl group (or a pyridinediyl group), a pyrimidinyl group (or a pyrimidine group), a pyrazyl group (or a pyrazine group), a quinolyl group (or a quinoline group), an isoquinolyl group (or an isoquinoline group), a carbazolyl group (or a carbazole group), a 9-phenylcarbazolyl group, an acridinyl group (or an acrydine group), a quinazolyl group (or a quinazoline group), a quinoxalyl group (or a quinoxaline group), a 1,6-naphthyridinyl group, a 1,8-naphthyridinyl group and a porphyrin group (or a porphyrin ring); and
heteroaryl groups containing two or more heteroatoms (e.g., nitrogen and sulfur), such as a benzothiazolyl group (or a benzothiazole group).

There is no particular limitation on the substituent, with which the aryl group and the heteroaryl group can be substituted, as long as the objective cross-coupling reaction of the present invention can be performed.

Examples of the substituent include:
alkyl groups having 1 to 24 carbon atoms, for example, 1 to 18 carbon atoms, for example, 1 to 12 carbon atoms, for example, 1 to 8 carbon atoms (e.g., a methyl group, an ethyl group, an n-propyl group, an isopropyl group, an n-butyl group, an isobutyl group, a tert-butyl group, a pentyl group, a hexyl group, an octyl group, etc.),
alkoxy groups having 1 to 24 carbon atoms, for example, 1 to 18 carbon atoms, for example, 1 to 12 carbon atoms, for example, 1 to 8 carbon atoms (e.g., a methoxy group, an ethoxy group, an n-propoxy group, an isopropoxy group, an n-butoxy group, an isobutoxy group, a tert-butoxy group, a pentyloxy group, a hexyloxy group, an octyloxy group, etc.),
cycloalkyl groups having 3 to 24 carbon atoms, for example, 3 to 18 carbon atoms, for example, 3 to 12 carbon atoms, for example, 3 to 8 carbon atoms (e.g., a cyclopropyloxy group, a cyclobutyloxy group, a cyclopentyloxy group, a cyclohexyloxy group, etc.),
alkenyl groups having 1 to 24 carbon atoms, for example, 1 to 18 carbon atoms, for example, 1 to 12 carbon atoms, for example, 1 to 8 carbon atoms (e.g., an ethenyl group, a propenyl group, a butenyl group, a pentenyl group, a hexenyl group, an octenyl group, etc.),
alkynyl groups having 1 to 24 carbon atoms, for example, 1 to 18 carbon atoms, for example, 1 to 12 carbon atoms, for example, 1 to 8 carbon atoms (e.g., an ethynyl group, a propynyl group, a butynyl group, a pentynyl group, a hexynyl group, an octynyl group, etc.),
aryl groups having 5 to 24 carbon atoms, for example, 5 to 18 carbon atoms, for example, 5 to 12 carbon atoms, for example, 5 to 8 carbon atoms (e.g., a phenyl group, a naphthyl group, a biphenyl group, etc.),
aryloxy groups having 5 to 24 carbon atoms, for example, 5 to 18 carbon atoms, for example, 5 to 12 carbon atoms, for example, 5 to 8 carbon atoms (e.g., a phenoxy group, a naphthyloxy group, a biphenyloxy group, etc.),
heteroaryl groups having 4 to 24 carbon atoms, for example, 1 to 18 carbon atoms, for example, 1 to 12 carbon atoms, for example, 1 to 8 carbon atoms (e.g., a thiophenyl group, a furanyl group, a carbazole group, a benzothiophenyl group, a benzofuranyl group, an indolyl group, a pyrrolyl group, a pyridyl group, etc.),
acyl groups having 1 to 24 carbon atoms, for example, 1 to 18 carbon atoms, for example, 1 to 12 carbon atoms, for example, 1 to 8 carbon atoms (e.g., an acetyl group, a propionyl group, a butanoyl group, a pentanoyl group, a heptanoyl group, a group in which a carbonyl group included in the acyl group is substituted with an ester group or an amide group, etc.),
amino groups having 1 to 24 carbon atoms, for example, 1 to 18 carbon atoms, for example, 1 to 12 carbon atoms, for example, 1 to 8 carbon atoms (e.g., a diphenylamino group, a dimethylamino group, etc.), and
fluorine (including partial fluorine substitution and complete fluorine substitution), cyano groups and nitro groups.

In the embodiment of the present invention, the aromatic compound (2-5) having a thiol group is more specifically selected, for example, from the general formula (II-5):

HS-(Cₚ H_{2 p} )-A⁶

wherein A⁶ is selected from an optionally substituted aryl group and an optionally substituted heteroaryl group, and p is an integer of 0 to 20.

In formula (II-5), p is an integer of 0 to 20, p is preferably 10 or less, and more preferably 4 or less. A⁵ is not particularly limited as long as it can be selected from an optionally substituted aryl group and an optionally substituted heteroaryl group, and a cross-coupling reaction can be performed.

The optionally substituted aryl group and the optionally substituted heteroaryl group can include the above-mentioned optionally substituted aryl group and optionally substituted heteroaryl group.

In formula (II-5), the substituent of the optionally substituted aryl group and the optionally substituted heteroaryl group can be, for example, the above-mentioned substituent.

Further, the substituent may be further substituted with the above-mentioned substituent.

It is possible to more specifically exemplify, as A⁶ of the aromatic compound (2-5) having a thiol group, for example, the following groups:
naphthyl groups such as a naphthyl group, an aryl (e.g., phenyl) naphthyl group, a naphthyl group having an alkylene (e.g., ethylene) crosslink, and a naphthyl group having an arylene (e.g., phenylene) crosslink;
phenanthrenyl groups;
anthracenyl groups such as an anthracenyl group, an aryl (e.g., phenyl) anthracenyl group, a diaryl (e.g., dinaphthyl) anthracenyl group, and a diarylboryl (e.g., bis(trialkylphenyl)boryl) anthracenyl group;
pyrenyl groups such as a pyrenyl group, alkyl (e.g., t-butyl) pyrenyl group;
biphenyl groups such as a biphenyl group, and a biphenyl group having an alkylene (e.g., propyrene, isopropyrene) crosslink;
terphenyl groups such as terphenyl group, tetraaryl (e.g., tetraphenyl)terphenyl group;
triphenylenyl groups;
phenyl groups such as a 2-aryl (e.g., phenyl) ethenylphenyl group, a 1,2,2-triaryl (e.g., triphenyl) ethenylphenyl group, a 2-aryl (e.g., phenyl) ethynylphenyl group, a phenyl group, an alkyl (e.g., methyl) phenyl group, a dialkyl (e.g., dimethyl) phenyl group, an alkoxy (e.g., methoxy) phenyl group, a dialkylamino (e.g., dimethylamino) phenyl group, a diaryl (e.g., diphenyl) aminophenyl group, a perfluoroalkyl (e.g., trifluoromethyl) phenyl group, an alkyl (e.g., ethyl) oxycarbonylphenyl group, and an alkanoyl (e.g., acyl) phenyl group;
aryl (e.g., phenyl) carbazolyl groups;
anthracene-9.10-dione groups, and
thienyl groups and aryl (e.g., phenyl) thienyl groups.

It is possible to use, as the aromatic compound (2-5) having a thiol group, commercially available compounds.

The method for producing a mono-cross-coupled aromatic compound (3-1) of the embodiment of the present invention comprises:
<3> performing a cross-coupling reaction of the aromatic compound (1) having at least two leaving groups with the compound (2) in the presence of a palladium catalyst and a base, in the absence of a solvent.

In the embodiment of the present invention, the palladium catalyst is not particularly limited as long as it is usually used in a cross-coupling reaction, and the objective cross-coupling reaction of the present invention between the above-mentioned aromatic compound (1) having at least two leaving groups and compound (2) can be progressed in the absence of a solvent.

In the embodiment of the present invention, examples of the palladium catalyst include divalent palladium compounds such as palladium(II) acetate, palladium(II) chloride, palladium(II) bromide, palladium(II) acetylacetonate, dichlorobis(benzonitrile)palladium(II), dichlorobis(acetonitrile)palladium(II), dichlorobis(triphenylphosphine)palladium(II), dichlorotetraamminepalladium(II), dichloro(cycloocta-1,5-diene)palladium(II) and palladium trifluoroacetate(II); and zero-valent palladium compounds such as tris(dibenzilidenacetone)dipalladium(0), tris(dibenzilidenacetone)dipalladium chloroform complex(0) and tetrakis(triphenylphosphine)palladium(0).

The palladium catalysts can be used alone or in combination, respectively.

It is possible to use, as the palladium catalyst, commercially available products.

The palladium catalyst can be allowed to coexist with a coordinating compound such as a phosphine compound. The phosphine compound is not particularly limited as long as it is used in the objective cross-coupling reaction of the present invention.

It is possible to exemplify, as the phosphine compound, for example, arylphosphines such as triphenylphosphine, tri(o-tolyl)phosphine and tri(mesityl)phosphine; and alkylphosphines such as tri(tert-butyl)phosphine, tri(cyclohexyl)phosphine and tri(isopropyl)phosphine.

It is also possible to exemplify, as the phosphine compound, for example, arylalkylphosphines having both an aryl group and an alkyl group, such as DavePhos (see the following formula), tBuBrettPhos (see the following formula) and tBuXPhos (see the following formula). The alkylphosphines can include arylalkylphosphines. Further, the alkyl group includes a cycloalkyl group.

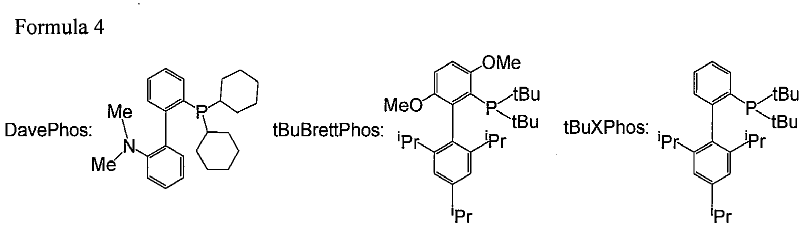

The phosphine compound preferably includes alkylphosphines.

The phosphine compounds can be used alone or in combination, respectively.

It is possible to use, as the phosphine compound, commercially available products.

When two cross-coupling reactions are performed, a phosphine compound having suppressed reactivity, for example, tBuXPhos or tBuBrettPhos is preferable in the first cross-coupling reaction.

In the second cross-coupling reaction, a phosphine compound having higher reactivity, for example, DavePhos is preferable.

In the embodiment of the invention, the base is not particularly limited as long as it is used in a cross-coupling reaction, and the objective cross-coupling reaction of the present invention between the above-mentioned aromatic compound (1) having at least two leaving groups and compound (2) can be progressed in the absence of a solvent.

It is possible to exemplify, as the base, for example:
inorganic bases such as sodium hydroxide, potassium hydroxide, sodium carbonate, potassium carbonate, rubidium carbonate, cesium carbonate, potassium phosphate, sodium phosphate, potassium fluoride and cesium fluoride;
alkali metal alkoxides such as sodium methoxide, sodium ethoxide, potassium methoxide, potassium ethoxide, lithium tert-butoxide, sodium tert-butoxide and potassium tert-butoxide; and
organic bases such as triethylamine, tributylamine, pyridine, diazabicycloundecene and diazabicyclononene.

The bases can be used alone or in combination, respectively.

It is possible to use, as the base, commercially available products.

In the embodiment of the present invention, the cross-coupling reaction is preferably performed in the presence of a compound having a carbon-carbon double bond or a carbon-carbon triple bond. The compound having a carbon-carbon double bond or a carbon-carbon triple bond is a compound having at least one carbon-carbon double bond or at least one carbon-carbon triple bond, and may be either chain or linear (excluding aromatic), and is not particularly limited as long as the objective cross-coupling reaction of the present invention between the above-mentioned aromatic compound (1) having at least two leaving groups and compound (2) can be progressed in the absence of a solvent. The above-mentioned aromatic compound (1) having at least two leaving groups, compound (2), palladium catalyst, coordinating compound and compound corresponding to the base are not included in the compound having a carbon-carbon double bond or a carbon-carbon triple bond.

The compound having a carbon-carbon double bond or a carbon-carbon triple bond preferably has 5 to 24 carbon atoms to promote the cross-coupling reaction. The number of carbon atoms of the compound having a carbon-carbon double bond or a carbon-carbon triple bond can be 5 to 18, 5 to 12, 6 to 10, or 6 to 8.

Examples of the chain compound having one carbon-carbon double bond include hexene, heptene, octene, nonene, decene and the like.

Examples of the cyclic compound having one carbon-carbon double bond include cyclohexene, cycloheptene, cyclooctene, cyclodecene and the like.

Examples of the chain compound having two carbon-carbon double bonds include hexadiene, heptadiene, octadiene, nonadiene, decadiene and the like.

Examples of the cyclic compound having two carbon-carbon double bonds include cyclohexadiene, cycloheptadiene, cyclooctadiene, cyclodecadiene and the like.

Examples of the chain compound having one carbon-carbon triple bond include hexyne, heptyne, octyne, decyne and the like.

Examples of the cyclic compound having one carbon-carbon triple bond include cyclooctyne, cyclodecyne and the like.

The compound having a carbon-carbon double bond or a carbon-carbon triple bond is preferably chain or monocyclic.

When two cross-coupling reactions are performed, the compound having a carbon-carbon double bond or a carbon-carbon triple bond is preferably used in the first cross-coupling reaction, and may be used or not in the second cross-coupling reaction. The compound having a carbon-carbon double bond or a carbon-carbon triple bond added in the first reaction, and the rest can also be used in the second reaction.

In the embodiment of the present invention, the cross-coupling reaction of the aromatic compound (1) having at least two leaving groups with the compound (2) can be performed by mixing (preferably shaking) in the absence of a solvent, in the presence of the palladium catalyst, the base and the compound having a carbon-carbon double bond or a carbon-carbon triple bond.

In the present disclosure, solvent-free usually means that the solvent usually used in a cross-coupling reaction (e.g., aromatic solvents such as benzene, toluene, xylene and mesityrene; ether-based solvents such as diethyl ether, diisopropyl ether, dibutyl ether, tetrahydrofuran, dimethoxyethane and 1,4-dioxane; alcohol-based solvents such as methanol, ethanol and t-butanol; and polar solvents such as acetonitrile, dimethylformamide and dimethylacetamide) is used in an amount of up to 0.2 mL or less, 0.1 mL or less, 0.05 mL or less, 0.02 mL or less, or 0.01 mL or less, per 1 mmol of the total of the compound (1) and compound (2), and that the reaction is performed without using the solvent, substantially and actively.

Generally, in the cross-coupling reaction, 1 to 2 mL of the solvent is used per 1 mmol of the total of reaction raw materials, so that the amount of the solvent used is obviously small in the embodiment of the present invention. In the embodiment of the present invention, the reaction raw materials can usually exist in a solid state without being partially dissolved in the solvent at the start of the reaction, and in some cases, without being completely dissolved in the solvent.

The cross-coupling reaction (mixing temperature) can be usually performed at room temperature (e.g., 5 to 40°C), and can be performed by heating as appropriate.

As the mixing method, any mixable method such as shaking, rubbing, pressing, dispersing, kneading and crushing may be used.

Mixing can be performed using, as such a device, for example:
crushers such as a ball mill, a rod mill, a jet mill and a SAG mill;
grinders such as a rotary stone mill and a bud crusher;
(horizontal axis rotation) container rotation type mixers such as horizontal cylindrical type, V type, double cone type, square cube type, S type and continuous V type mixers;
container rotation type mixers (with baffle plate blade) such as horizontal cylinder type, V type, double cone type and ball mill type mixers;
(rotary vibration) container rotary type mixers such as locking type and cross-rotary type mixers;
(horizontal axis rotation) fixed container type mixers such as ribbon type, paddle type, single shaft rotor type and bug mill type mixers;
(vertical axis rotation) fixed container type mixers such as ribbon type, screw type, planet type, turbine type, high-speed fluid type, rotating disk type and Marler type mixers;
(vibration) fixed container type mixers such as a vibration mill type mixer and a sieve;
(fluidized) fluid motion type mixers such as non-uniform fluidized bed, swirl fluidized bed, riser pipe type and jot pump type mixers; and
(gravity) fluid motion type mixers such as a gravity type mixer and a static mixer. As long as the cross-coupling reaction proceeds, the method and the apparatus used are not particularly limited. Regarding the mixing device, it is possible to refer to, for example, Sakashita "Powder Mixing Process Technology", Coloring Material, 77 (2), 75-85 (2004), Table 5 and FIG. 9.

The mixing rate can also be appropriately selected.

The mixing time can also be appropriately selected. In the embodiment of the present invention, the mixing time can be, for example, 15 minutes or more, 30 minutes or more, 45 minutes or more, 60 minutes or more, 2 hours or less, 3 hours or less, 5 hours or less, or 10 hours or less.

The equivalent ratio of the compound (2) to the aromatic compound (1) having at least two leaving groups (compound (2)/aromatic compound (1) having at least two leaving groups) is not particularly limited as long as it is the equivalent ratio which can allow the cross-coupling reaction to proceed, and may be, for example, 0.6 to 1.6, 0.8 to 1.4, or 1.0 to 1.2. It is preferable to react at almost the same equivalent.

The equivalent of the base is not particularly limited as long as it is the amount which can allow the cross-coupling reaction to proceed, and can be, for example, 0.5 or more, 0.8 or more, 1.0 or more, 1.2 or more, 1.4 or more, 3 or less, 4 or less, 5 or less, or 10 or less, based on the equivalent of the aromatic compound (1) having at least two leaving groups.

The amount of the palladium catalyst is not particularly limited as long as it is the amount which can allow the cross-coupling reaction to proceed. For example, the amount can be 0.5 mol% or more, 0.1 mol% or more, 0.5 mol% or more, 1 mol% or more, 25 mol% or less, 20 mol% or less, 15 mol% or less, or 10 mol% or less, based on the mol number (100%) obtained by multiplying the mol number of the aromatic compound (1) having at least two leaving groups by the valence of the compound (1).

When the phosphine compound is allowed to coexist with the palladium catalyst, a molar ratio of the phosphine compound to the palladium catalyst (phosphine compound/palladium catalyst) is not particularly limited as long as it is the molar ratio which can allow the cross-coupling reaction to proceed, and may be, for example, 10/1 to 1/10, 5/1 to 1/5, 3/1 to 1/3, or 2/1 to 1/2.

The amount of the compound having a carbon-carbon double bond or a carbon-carbon triple bond added is not particularly limited as long as it is the amount which can allow the cross-coupling reaction to proceed. For example, it is possible to add in the amount of 0.01 to 3 microL/mg, 0.05 to 1 microL/mg, or 0.1 to 0.5 microL/mg, based on the total mass of all reaction raw materials added to perform the cross-coupling reaction (e.g., the dihalogenated aromatic compound (1), the compound (2), the palladium catalyst, the base, and the phosphine compound when the phosphine compound is added).

It is possible to additionally use a conventionally known accelerator for cross-coupling reaction in the cross-coupling reaction of the present disclosure. Examples of such accelerator include water and the like. The amount of the additional accelerator is not particularly limited as long as it is the amount which can allow the cross-coupling reaction to proceed.

The mono-cross-coupled aromatic compound (or mono-coupled product) (3-1) can be appropriately purified. The purification method is not particularly limited as long as the mono-cross-coupled aromatic compound (3-1) can be purified. It is possible to use, as the purification method, for example, a conventional method such as recrystallization or column chromatography.

In the embodiment of the present invention, there is provided a method for producing a mono-cross-coupled aromatic compound (3-1), which is obtained by a reaction of an aromatic compound (1) having at least two leaving groups with an aromatic boronic acid (2-1), an aromatic amino compound (2-2), a diboronic acid ester (2-3), an aromatic compound (2-4) having a hydroxyl group or an aromatic compound (2-5) having a thiol group, wherein the mono-cross-coupled aromatic compound (3-1) is selected from mono-cross-coupled aromatic compounds represented by the following general formulas (III-1-1) to (III-1-5):

X_{n - 1} -A¹ -A² : III-1-1

X_{n - 1} -A¹ -N(A³)A⁴ : III-1-2

X_{n - 1} -A¹ -B(OR³)OR⁴: III-1-3

X_{n - 1} -A¹ -O-(Cm H_{2 m} )-A⁵ : III-1-4

X_{n - 1} -A¹ -S-(Cₚ H_{2 p} )-A⁶ : 111-1-5

wherein A¹ to A⁶, R⁵ to R⁶, m, p and X are the same as defined in general formulas (I) and (11-1) to (II-5).

In the embodiment of the present invention, there is provided a method for producing a mono-cross-coupled aromatic compound (3-1), wherein a melting point of the mono-cross-coupled aromatic compound (3-1) is higher than that of the aromatic compound (1) having at least two leaving groups, and a difference between the melting point of the aromatic compound (1) having at least two leaving groups and that of the mono-cross-coupled aromatic compound (3-1) is 10°C or higher.

The difference between the melting point of the aromatic compound (1) having at least two leaving groups and that of the mono-cross-coupled aromatic compound (3-1) is, for example, 10°C or higher, preferably 20°C or higher, more preferably 30°C or higher, still more preferably 40°C or higher; particularly preferably 50°C or higher, and extremely preferably 60°C or higher.

When the melting point of the mono-cross-coupled aromatic compound (3-1) is higher than that of the aromatic compound (1) having at least two leaving groups, it is preferred since the reaction rate of the mono-cross-coupled aromatic compound (2) can be decreased compared to that of the aromatic compound (1) having at least two leaving groups.

When the difference between the melting point of the aromatic compound (1) having at least two leaving groups and that of the mono-cross-coupled aromatic compound (3-1) is 10°C or higher, it is preferred since the difference in the reaction rate between the aromatic compound (1) having at least two leaving groups and the mono-cross-coupled aromatic compound (3-1) can be more increased.

In the embodiment of the present invention, there is provided a method for producing a mono-cross-coupled aromatic compound (3-1), wherein a melting point of the mono-cross-coupled aromatic compound (3-1) is higher than that of the aromatic compound (1) having at least two leaving groups, and a reaction temperature of a cross-coupling reaction is between the melting point of the mono-cross-coupled aromatic compound (3-1) and that of the aromatic compound (1) having at least two leaving groups.

When the reaction temperature of the cross-coupling reaction is between the melting point of the aromatic compound (1) having at least two leaving groups and that of the mono-cross-coupled aromatic compound (3-1), it is thought that the aromatic compound (1) having at least two leaving groups is liquid, and the mono-cross-coupled aromatic compound (3-1) is in a non-liquid state that has lost fluidity, and preferably solid, in the reaction system. In this case, it is preferred that the aromatic compound (1) having at least two leaving groups and the mono-cross-coupled aromatic compound (3-1) can differ more clearly in reactivity.

The method for producing a mono-cross-coupled aromatic compound (3-1) of the embodiment of the present invention can comprise producing a solid mono-cross-coupled aromatic compound (3-1) from a liquid aromatic compound (1) having at least two leaving groups in the reaction system.

In the embodiment of the present invention, according to the method for producing a mono-cross-coupled aromatic compound (3-1), the mono-cross-coupled aromatic compound (3-1) may be in a state that has lost fluidity, and is not necessarily crystalline. The solid mono-cross-coupled aromatic compound (3-1) may be produced in the reaction system, may be produced from the initial stage of the reaction, and may increase as the reaction proceeds.

In a method for producing a mono-cross-coupled aromatic compound (3-1) of the embodiment of the present invention, there is provided a method for producing a mono-cross-coupled aromatic compound (3-1), wherein, regarding the product (the di-cross-coupled aromatic compound) (3-2) which underwent two cross-coupling reactions and is produced simultaneously when the mono-cross-coupled aromatic compound (3-1) is obtained, a ratio of the mono-cross-coupled aromatic compound (3-1) to the di-cross-coupled aromatic compound (3-2) (mono-cross-coupled aromatic compound (3-1)/di-cross-coupled aromatic compound (3-2)) is more than 1.

In the embodiment of the present invention, the ratio of the mono-cross-coupled aromatic compound (3-1) to the di-cross-coupled aromatic compound (3-2) produced when the mono-cross-coupled aromatic compound (3-1) is obtained (mono-cross-coupled aromatic compound (3-1)/di-cross-coupled aromatic compound (3-2)) is preferably more than 1, more preferably more than 2, still more preferably more than 3, and particularly preferably more than 4.

When mono-cross-coupled aromatic compound (3-1)/di-cross-coupled aromatic compound (3-2) is more than 1, it is preferred since the mono-cross-coupled aromatic compound (3-1) is obtained in preference to the di-cross-coupled aromatic compound (3-2).

In a preferred aspect of the present invention, there is provided a method for producing a di-cross-coupled aromatic compound (3-2) which underwent two cross-coupling reactions, the method comprising:
including the method for producing a mono-cross-coupled aromatic compound (3-1), and
allowing the mono-cross-coupled aromatic compound (3-1) to undergo a second cross-coupling reaction.

The mono-cross-coupled aromatic compound (3-1) which can be produced in the present disclosure can be subjected to a cross-coupling reaction and used to produce a di-cross-coupled aromatic compound (3-2) which underwent two cross-coupling reactions. The second cross-coupling reaction may be different from the first reaction, and the compound (2) used for the second reaction may be different from the compound (2) in the first reaction. Therefore, in the embodiment of the present invention, the aromatic compound (1) having at least two leaving groups can be more selectively reacted with two different types of compounds (2) to produce such di-cross-coupled aromatic compound (3-2).

In the embodiment of the present invention, there is provided a method for producing a di-cross-coupled aromatic compound (3-2) which underwent (or was subjected to) two cross-coupling reactions, the method comprising allowing a mono-cross-coupled aromatic compound (3-1) to undergo a second cross-coupling reaction in the absence of a solvent.

The second cross-coupling reaction is preferably performed in the absence of a solvent.

In the embodiment of the present invention, there is provided a method for producing a di-cross-coupled aromatic compound (3-2), wherein a compound (2) used for a first cross-coupling reaction and a compound (2) used for a second cross-coupling reaction are different.

In the embodiment of the present invention, there is provided a method for producing a di-cross-coupled aromatic compound (3-2), wherein the first mono-cross-coupled aromatic compound (3-1) directly undergoes a second cross-coupling reaction without purification.

When the second cross-coupling is performed, the mono-cross-coupled aromatic compound (3-1) may be used with or without purification. When the mono-cross-coupled aromatic compound (3-1) is not purified, the di-cross-coupled aromatic compound (3-2) may be produced in one pot.

As mentioned above, the reaction conditions of the first coupling reaction and the second cross-coupling reaction are not particularly limited, and can be appropriately adjusted as mentioned above, respectively.

### [Examples]

The present invention will be described below by way of Examples and Comparative Examples. However, these Examples are merely an embodiment of the invention and the present invention is in no way limited by these Examples.

The compounds used in Examples were specifically exemplified in the following Examples.

Regarding the compounds used in the present invention, such as the aromatic compound (1) having at least two leaving groups, the compound (2) which is capable of undergoing a cross-coupling reaction with the aromatic compound (1) having at least two leaving groups, the palladium compound, the phosphine compound, and the compound having a carbon-carbon double or triple bond, commercially available products were directly used without purification, unless indicated otherwise.

The cross-coupling reaction was performed at room temperature using a ball mill, Model MM400, manufactured by Retsch Co., Ltd. (changed company name to Verder Scientific Co., Ltd.).

### Example 1

In a 1.5 mL stainless steel ball mill jar containing a stainless steel ball having a diameter of 3 mm, 1,2-dibromobenzene (1a) (0.30 mmol, 1.0 equiv), [4-(dimethylamino)phenyl]boronic acid (2a) (0.36 mmol, 1.2 equiv) and palladium acetate (0.009 mmol, 3 mol %) were charged under the air. The ball mill jar was transferred to a glove box, and then tBuXPhos (0.0135 mmol, 4.5 mol %) and CsF (0.9 mmol, 3.0 equiv) were added under argon atmosphere. The ball mill jar was removed from the glove box, and then 1,5-cyclooctadiene (4a) (0.20 microL/mg based on the total mass of 1,2-dibromobenzene, [4-(dimethylamino)phenyl]boronic acid, palladium acetate, tBuXPhos and CsF) and water (20 microL) were added again under the air. A lid of the ball mill jar was closed and the ball mill jar was attached to a ball mill (Model MM400, manufactured by Retsch Co., Ltd.), followed by shaking for 99 minutes and further stirring (25 Hz). After completion of the reaction, the reaction mixture was passed through short silica gel column chromatography with ethyl acetate to remove the catalyst and the inorganic salt. After removing ethyl acetate by an evaporator, a cross-coupled product was isolated by purification using silica gel column chromatography (yield of 78%). As a result of analysis by ¹H-NMR, a ratio of a mono-cross-coupled aromatic compound (3-1a) to a di-cross-coupled aromatic compound (3-2a) was 92:8.

The results of Example 1 are also shown in Table 1.

The same reaction was performed using an organic solvent (toluene) at the concentration of 0.1 mol/L at 50°C for 24 hours and, as a result, the yield was 43%, and a ratio of a mono-cross-coupled aromatic compound (3-1a) to a di-cross-coupled aromatic compound (3-2a) was 30:70. The results are shown in()^{sol} of Table 1.

### Examples 2 to 8

In the same manner as in Example 1, except that 1,2-dibromobenzene (1a) and/or [4-(dimethylamino)phenyl]boronic acid (2a) of Example 1 were changed to compounds mentioned in Tables 1 and 2 (and except that 2.0 equiv of [4-(methoxy)phenyl]boronic acid (2b) was used in Example 2), the reactions of Examples 2 to 8 were performed to obtain cross-coupled reaction products. The results of Examples 2 to 8 and the results obtained by using corresponding organic solvent are shown in Table 1 and 2.

### Examples 11 to 14

In the same manner as in Example 1, except that 1,2-dibromobenzene (1a) and/or [4-(dimethylamino)phenyl]boronic acid (2a) of Example 1 were changed to compounds mentioned in Table 3, and that 0.40 microL/mg of 1,5-cyclooctadiene was used (and except that 0.10 microL/mg of 1,5-cyclooctadiene was used in Example 12, and 0.80 microL/mg of 1,5-cyclooctadiene was used in Example 14, and shaking was performed for 3 hours), the reactions of Examples 11 to 14 were performed to obtain cross-coupled reaction products. The results of Examples 11 to 14 and the results obtained by using corresponding organic solvent are shown in Table 3.

### Examples 15 to 18

In the same manner as in Example 1, except that 1,2-dibromobenzene (1a) and/or [4-(dimethylamino)phenyl]boronic acid (2a) of Example 1 were changed to compounds mentioned in Table 4, and that 0.40 microL/mg of 1,5-cyclooctadiene was used and t-Bu3P was used as the phosphorus compound, the reactions of Examples 15 to 18 were performed to obtain cross-coupled reaction products. The results of Examples 15 to 18 and the results obtained by using corresponding organic solvent are shown in Table 4. In Example 18, 2 equiv of a boronic acid (2b) was used and tBuXPhos was used as the phosphorus compound. The results of Examples 15 to 18 and the results obtained by using corresponding organic solvent are shown in Table 3.

### Examples 21 to 24

Examples 21 to 24 show Production Examples of a di-cross-coupled aromatic compound (3-2) in which two cross-coupling reactions are different, based on the results of Examples 1 to 17.

In Example 21, in the same manner as in Example 1, except that a mono-cross-coupled aromatic compound (3-1d) obtained in Example 4 was used, boronic acid (2i) and DavePhos were used, and 1,5-cyclooctadiene was not used, a second cross-coupling reaction was further performed to obtain a di-cross-coupled aromatic compound (3-2r). The total yield for the first and second cross-coupling reactions was 67%.

In Example 22, in the same manner as in Example 1, except that a mono-cross-coupled aromatic compound (3-1n) obtained in Example 15 was used, boronic acid (2j) and DavePhos were used, and 1,5-cyclooctadiene was not used, a second cross-coupling reaction was further performed to obtain a di-cross-coupled aromatic compound (3-2s). The total yield for the first and second cross-coupling reactions was 51%.

In Example 23, in the same manner as in Example 1, except that a mono-cross-coupled aromatic compound (3-1o) obtained in Example 16 was used, boronic acid (2i) and DavePhos were used, and 1,5-cyclooctadiene was not used, a second cross-coupling reaction was further performed to obtain a di-cross-coupled aromatic compound (3-2t). The total yield for the first and second cross-coupling reactions was 48%.

In Example 24, in the same manner as in Example 1, except that a mono-cross-coupled aromatic compound (3-1k) obtained in Example 13 was used, boronic acid (2k) and DavePhos were used, and 1,5-cyclooctadiene was not used, a second cross-coupling reaction further performed to obtain a di-cross-coupled aromatic compound (3-2u). The total yield for the first and second cross-coupling reactions was 42%.

The results of Examples 21 to 24 are shown in Table 5.

### Example 25

Example 25 shows Production Example of a di-cross-coupled aromatic compound (3-2) in which two different cross-coupling reactions are continuously performed in one pot.

In Example 25, in the same manner as in Example 1, except that the 1,2-dibromobenzene (1a) of Example 1 was changed to dibromothiophene (1f), boronic acid (2h) and t-BuBrettPhos were used, and purification was not performed, a first cross-coupling reaction was performed. Subsequently, in the same manner as in the first cross-coupling reaction, except that the crude product was not purified, boronic acid (2b) and DavePhos were used, and 1,5-cyclooctadiene was not used, a second cross-coupling reaction was further performed to obtain a di-cross-coupled aromatic compound (3-2v). The total yield for the first and second cross-coupling reactions was 54%.

The results of Example 25 are shown in Table 6.

### Observation of Reaction using Powder X-Ray Diffraction

The reaction of Example 5 was observed by powder X-ray diffraction (PXRD). FIG. 1 shows the observation of a coupling reaction of Example 5 by powder X-ray diffraction. In the crude product after the reaction, a new peak attributing to the mono-cross-coupled aromatic compound (3-1e) was mainly observed. This indicates that the reaction produces a mono-cross-coupled aromatic compound (3-1e), but not produces much di-cross-coupled aromatic compound (3-2e).

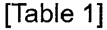

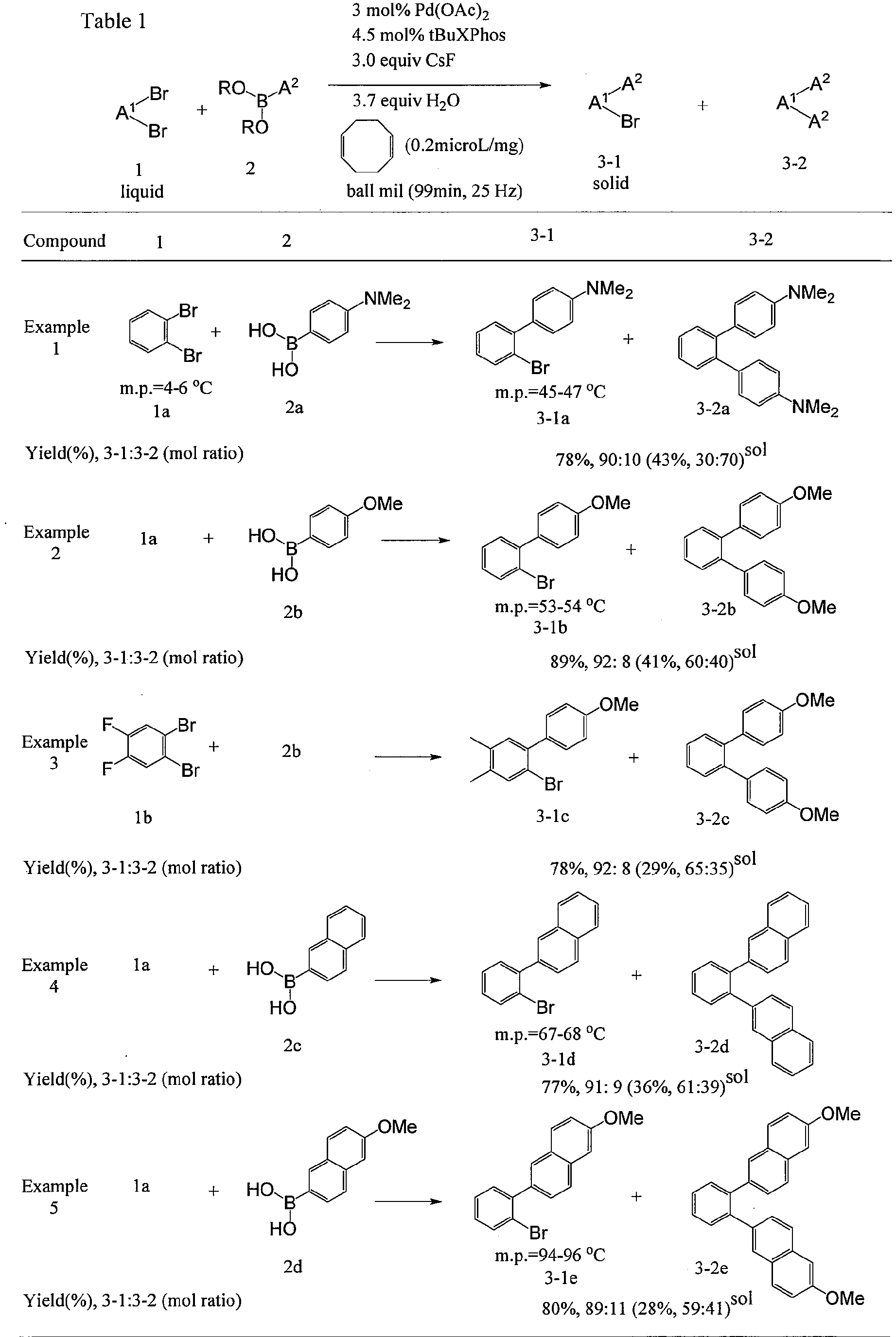

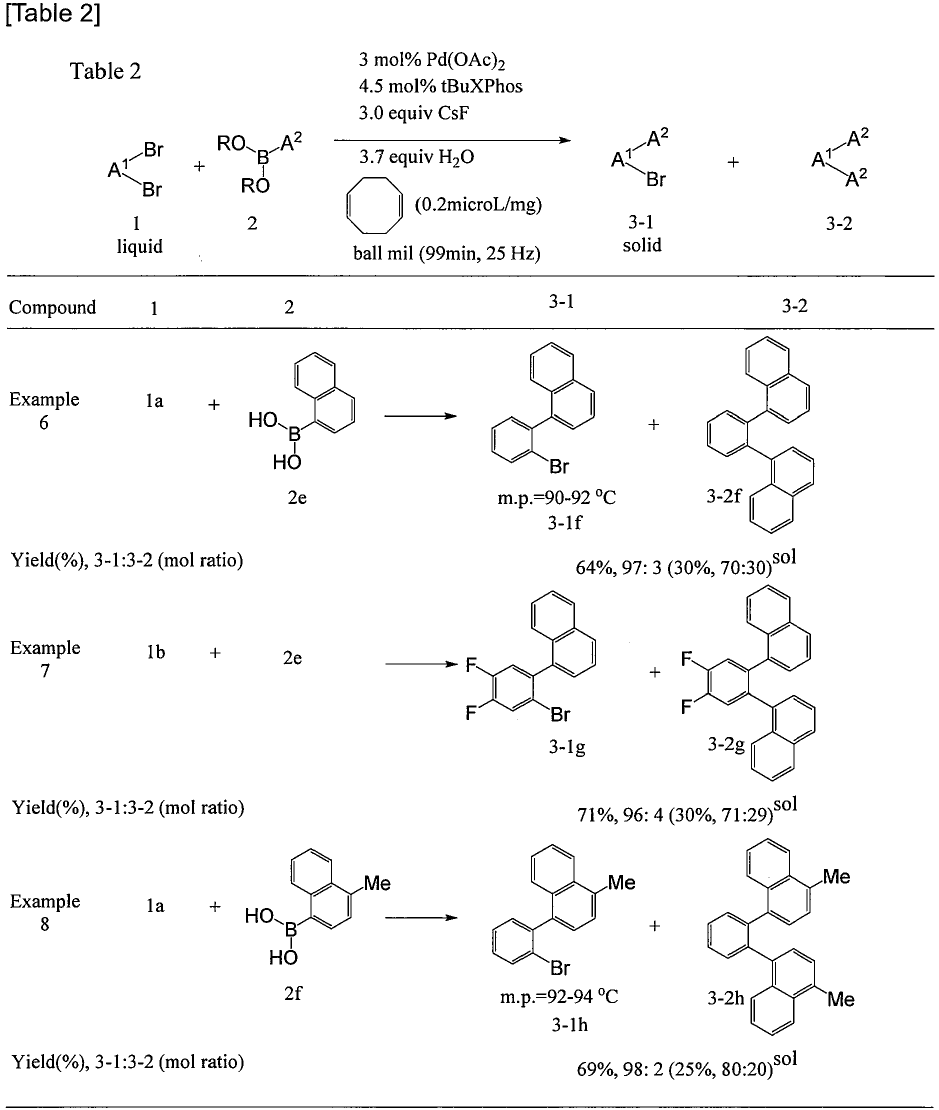

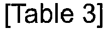

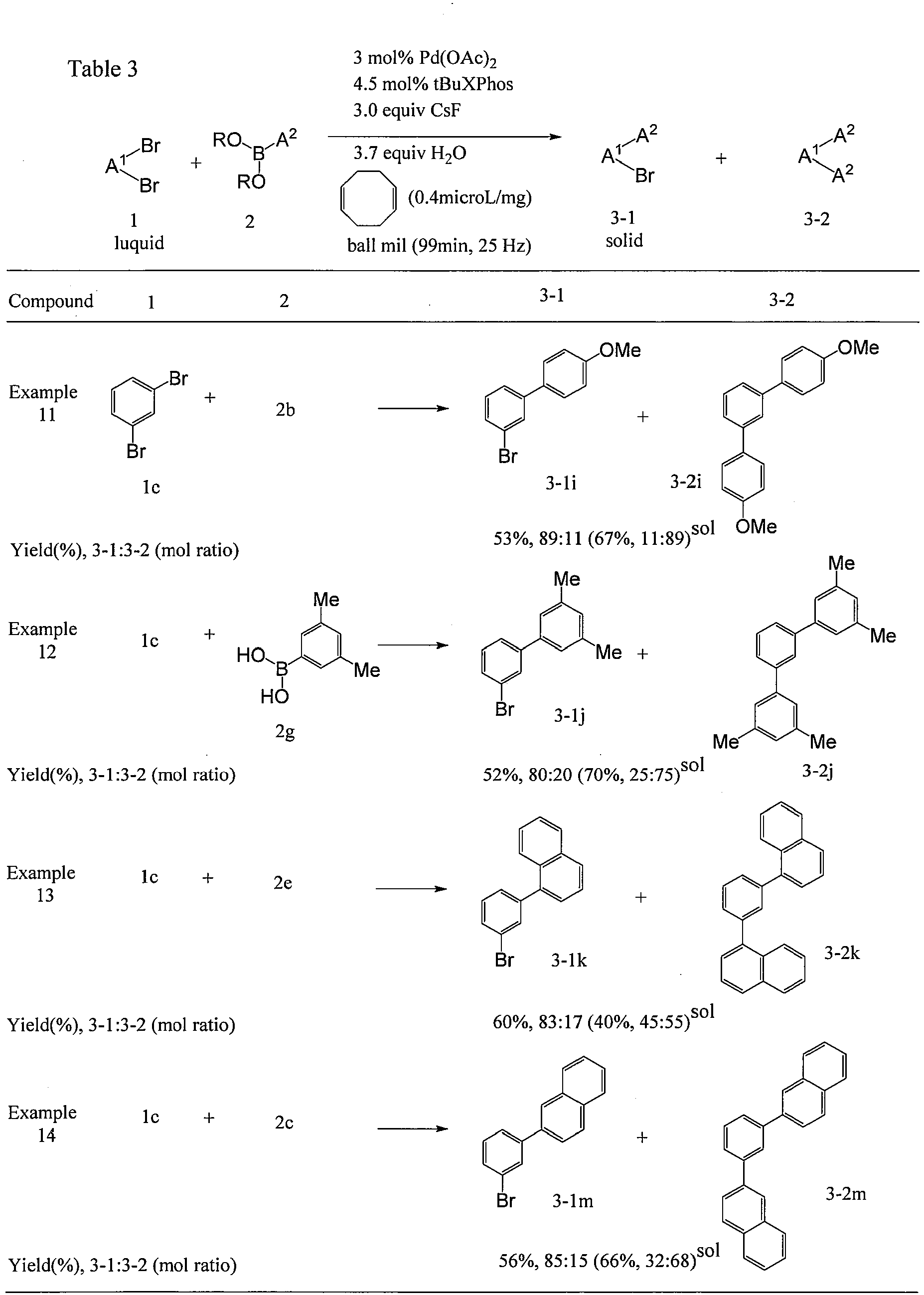

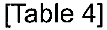

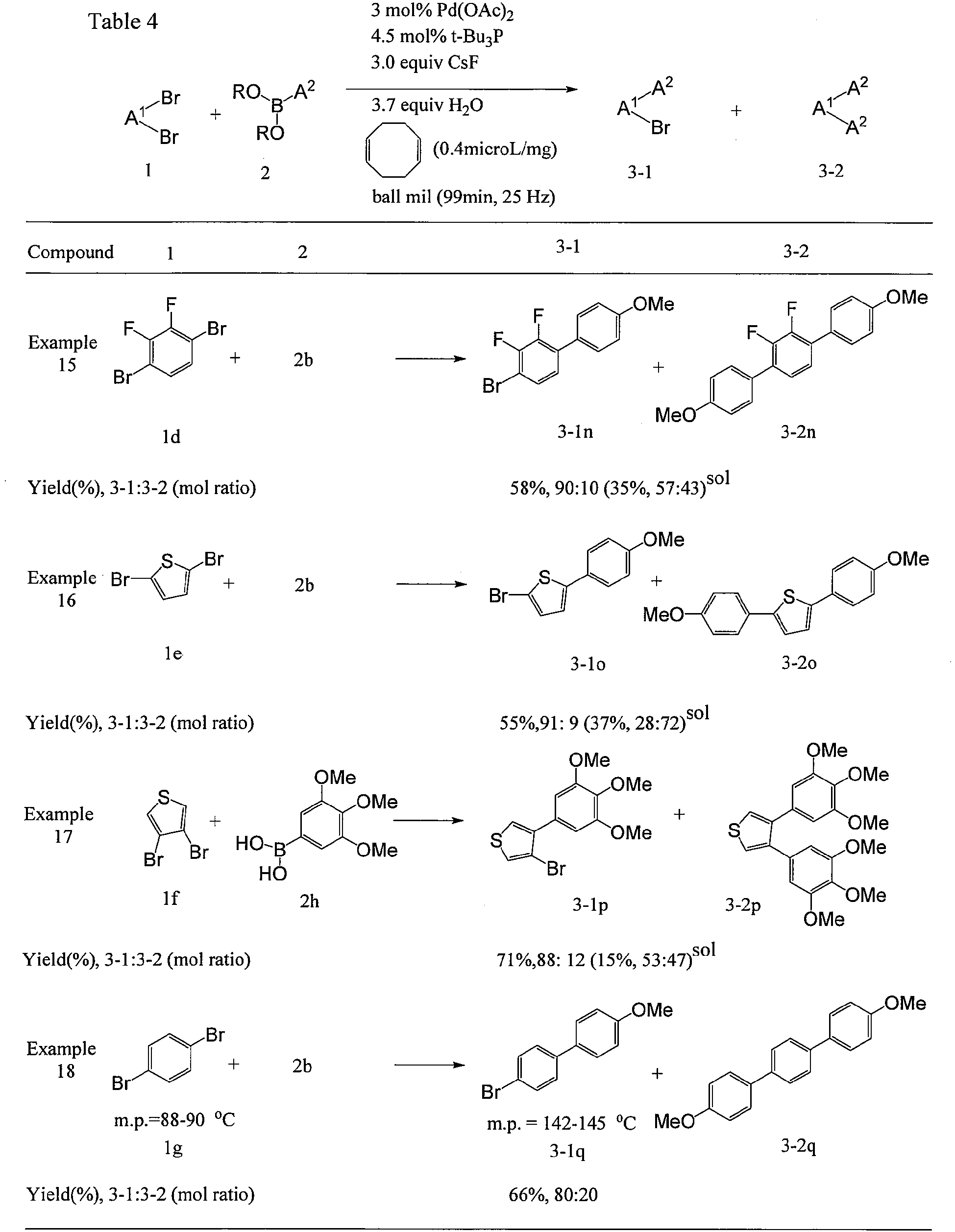

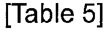

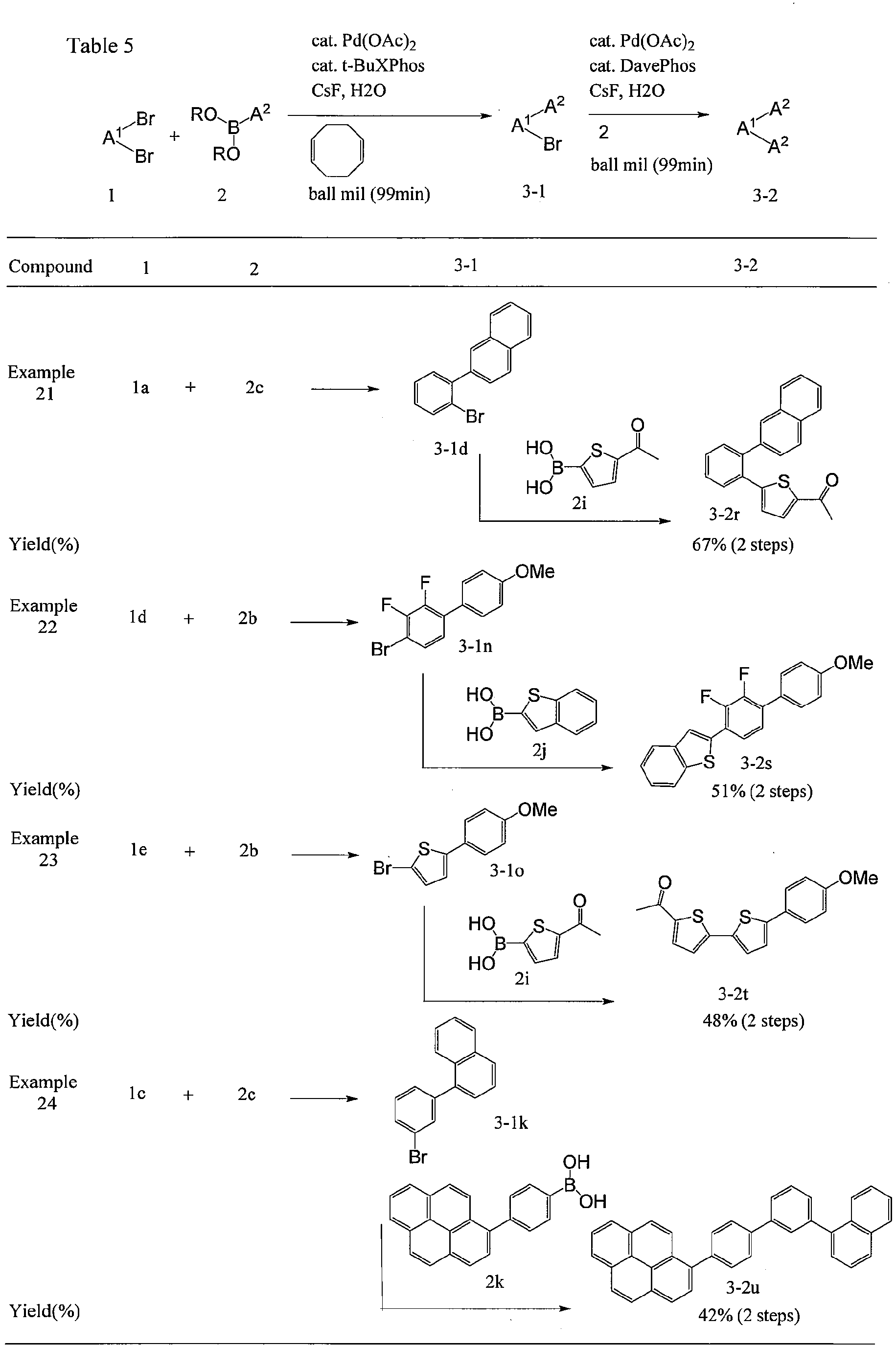

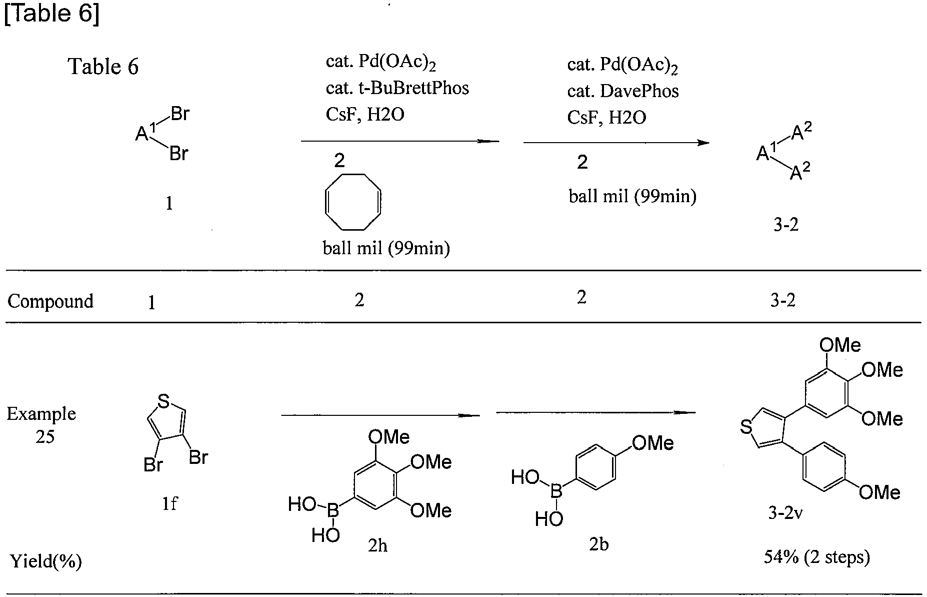

In the cross-coupling reactions of Examples 1 to 17, the aromatic compound (1) having at least two leaving groups is liquid at room temperature. The reaction between the aromatic compound (1) having at least two leaving groups and the compound (2) capable of undergoing a cross-coupling reaction proceeded well without using solvent to give much more mono-cross-coupled aromatic compound (3-1). Simultaneously, the di-cross-coupled aromatic compound (3-2), which underwent the cross-coupling reaction twice, was also produced, but was less than the mono-cross-coupling aromatic compound (3-1). Since all the mono-cross coupling aromatic compounds (3-1) are solid at room temperature, it is thought that the reaction rate decreased compared to the liquid aromatic compound (1) having at least two leaving groups due to diffusion limitation, which makes it difficult to produce the di-cross-coupled aromatic compound (3-2). The melting point of the aromatic compound (1a) having at least two leaving groups is 4 to 6°C. All the melting points of the mono-cross-coupled aromatic compounds (3-1a), (3-1b), (3-1d), (3-1e), (3-1f) and (3-1h) of Examples 1, 2, 4, 5, 6 and 8 were higher than the melting point of the aromatic compound (1a) having at least two leaving groups, as shown in Tables 1 and 2.

The results obtained by performing similar reaction using an organic solvent are shown in Tables 1 to 4. When the organic solvent is used, the di-cross-coupled aromatic compound (3-2) was obtained much more than the mono-cross coupling aromatic compound (3-1). This contrasts with the reaction without using a solvent in Examples 1 to 17.

In the cross-coupling reaction of Example 18, the aromatic compound (1) having at least two leaving groups is solid at room temperature. The reaction between the aromatic compound (1) having at least two leaving groups and the compound (2) capable of undergoing a cross-coupling reaction proceeded well without using solvent to give much more mono-cross-coupled aromatic compound (3-1). Simultaneously, the di-cross-coupled aromatic compound (3-2), which underwent the cross-coupling reaction twice, was also produced, but was less than the mono-cross-coupling aromatic compound (3-1). The mono-cross coupled aromatic compound (3-1) is solid at room temperature and has a higher melting point. Since the mono-cross coupling aromatic compound has a firmer solid structure, it is thought that the reaction rate decreased compared to the liquid aromatic compound (1) having at least two leaving groups, which makes it difficult to produce the di-cross-coupled aromatic compound (3-2).

The cross-coupling reaction of Examples 21 to 25 indicates that, when two cross-coupling reaction are performed, the di-cross-coupled aromatic compound (3-2) reacted with two different types of compounds (2) can be easily obtained in relatively satisfactory yield.

In Examples 21 to 24, a mono-cross-coupled aromatic compound (3-1) was obtained in the first reaction, and after purification, the mono-cross-coupled aromatic compound (3-1) was reacted with the compound (2) in the second reaction, which compound (2) is different from the compound (2) in the first reaction, to give a di-cross-coupled aromatic compound (3-2) reacted with two different types of compounds (2).

In Example 25, a mono-cross-coupled aromatic compound (3-1) was obtained in the first reaction, and then the mono-cross-coupled aromatic compound (3-1) was directly reacted with the compound (2) in the second reaction without purification, which compounds (2) is different from the compound (2) in the first reaction, to give a di-cross-coupled aromatic compound (3-2) reacted with two different types of compounds (2). Example 25 indicated that the di-cross-coupling aromatic compound (3-2) reacted with two types of compounds (2) can be obtained in one pot.

### [Industrial Applicability]

When using the production method of the embodiment of the present invention, a mono-cross-coupled aromatic compound (3-1) can be produced from an aromatic compound (1) having at least two leaving groups, more selectively and efficiently. Furthermore, in the embodiment of the present invention, the mono-cross-coupled aromatic compound (3-1) can be further cross-coupled to produce a di-cross-coupled aromatic compound (3-2).

### [Related Applications]

This application claims priority under Article 4 of the Paris Convention or Article 41 of the Japanese Patent Law on Japanese Patent Application No. 2020-35093 filed on March 2, 2020 in Japan, the disclosure of which is incorporated by reference herein.

## Claims

1. A method for producing a mono-cross-coupled aromatic compound (3-1) having one less leaving group than an aromatic compound (1) having at least two leaving groups, the method comprising:
preparing the aromatic compound (1) having at least two leaving groups;
preparing a compound (2) capable of undergoing a cross-coupling reaction selected from an aromatic boronic acid (2-1), an aromatic amino compound (2-2), a diboronic acid ester (2-3), an aromatic compound (2-4) having a hydroxyl group and an aromatic compound (2-5) having a thiol group; and
performing a cross-coupling reaction of the aromatic compound (1) having at least two leaving groups with the compound (2) in the presence of a palladium catalyst and a base, in the absence of a solvent.

2. The method for producing a mono-cross-coupled aromatic compound (3-1) according to claim 1, wherein the aromatic compound (1) having at least two leaving groups is a halogenated aromatic compound in which the leaving group is halogen, and the mono-cross-coupled aromatic compound (3-1) is a halogenated mono-cross-coupled aromatic compound.

3. The method for producing a mono-cross-coupled aromatic compound (3-1) according to claim 1 or 2, wherein the aromatic compound (1) having at least two leaving groups is represented by the following general formula (I):
A¹ -Xn
wherein A¹ is selected from an optionally substituted aromatic group and an optionally substituted heteroaromatic group, X is a leaving group selected from a halogeno group, a diazonium salt, trifluoromethanesulfonate and a carboxylic acid derivative, and n is an integer of 2 or more;
the aromatic boronic acid (2-1) is represented by the following general formula (II-1): 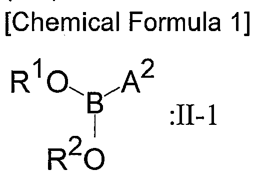 wherein R¹ to R² are each independently selected from hydrogen, an optionally substituted alkyl group and an optionally substituted aryl group, R¹ and R² may be bonded to each other, and A² is selected from an optionally substituted aryl group and an optionally substituted heteroaryl group;
the aromatic amino compound (2-2) is represented by the following general formula (II-2): 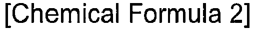 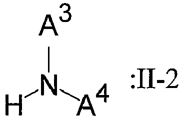 wherein A³ and A⁴ are each independently selected from hydrogen, an optionally substituted aryl group and an optionally substituted heteroaryl group, A³ and A⁴ are not simultaneously hydrogen, and A³ and A⁴ may be bonded to each other;
the diboronic acid ester (2-3) is represented by the following general formula (II-3): 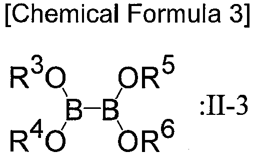 wherein R³ to R⁶ are each independently selected from hydrogen, an optionally substituted alkyl group and an optionally substituted aryl group, R³ and R⁴ may be bonded to each other, and R⁵ and R⁶ may be bonded to each other;
the aromatic compound (2-4) having a hydroxyl group is represented by the following general formula (II-4):
HO-(Cm H_{2 m} )-A⁵
wherein A⁵ is selected from an optionally substituted aryl group and an optionally substituted heteroaryl group, and m is an integer of 0 to 20; and
the aromatic compound (2-5) having a thiol group is represented by the following general formula (II-5):
HS-(Cₚ H_{2 p} )-A⁶
wherein A⁶ is selected from an optionally substituted aryl group and an optionally substituted heteroaryl group, and p is an integer of 0 to 20.

4. The method for producing a mono-cross-coupled aromatic compound (3-1) according to any one of claims 1 to 3, wherein the aromatic compound (1) having at least two leaving groups contains two leaving groups and an aromatic group,
two leaving groups are selected from a halogeno group, a diazonium salt, trifluoromethanesulfonate and a carboxylic acid derivative, and two leaving groups may be the same or different,
the aromatic group is selected from an optionally substituted aromatic group containing no heteroatom and an optionally substituted aromatic group containing a heteroatom,
the optionally substituted aromatic group containing no heteroatom includes a phenylene group, a naphthalenediyl group, an anthracenediyl group, a phenanthrenediyl group, a biphenyldiyl group, a terphenyldiyl group, a pyrenediyl group, a perylenediyl group and a triphenylenediyl group,
the optionally substituted aromatic group containing a heteroatom includes a sulfur-containing aromatic group; an oxygen-containing aromatic group; a nitrogen-containing aromatic group; and an aromatic group containing two or more heteroatoms,
the aromatic boronic acid (2-1) includes an aromatic boronic acid and an aromatic boronic acid ester, and contains, as an aromatic group, one selected from an optionally substituted aryl group and an optionally substituted heteroaryl group,
the optionally substituted aryl group includes a phenyl group, a naphthyl group, an anthracenyl group, a phenanthrenyl group, a biphenyl group, a terphenyl group, a pyrenyl group, a perylenyl group and a triphenylenyl group,
the optionally substituted heteroaryl group includes a sulfur-containing heteroaryl group; an oxygen-containing heteroaryl group; a nitrogen-containing heteroaryl group; and a heteroaryl group containing two or more heteroatoms,
the aromatic amino compound (2-2) can include up to two aromatic groups, and the aromatic group can be selected from an optionally substituted aryl group and an optionally substituted heteroaryl group,
the optionally substituted aryl group includes a phenyl group, a naphthyl group, an anthracenyl group, a phenanthrenyl group, a biphenyl group, a terphenyl group, a pyrenyl group, a perylenyl group and a triphenylenyl group,
the optionally substituted heteroaryl group includes a sulfur-containing heteroaryl group; an oxygen-containing heteroaryl group; a nitrogen-containing heteroaryl group; and a heteroaryl group containing two or more heteroatoms,
the diboronic acid ester (2-3) includes a diboronic acid alkyl ester, a diboronic acid alkylene glycol ester, a diboronic acid aryl ester, a diboronic acid arylene glycol ester and tetrahydroxydiborane,
the aromatic compound (2-4) having a hydroxyl group contains, as an aromatic group, one selected from an optionally substituted aryl group and an optionally substituted heteroaryl group,
the optionally substituted aryl group includes a phenyl group, a naphthyl group, an anthracenyl group, a phenanthrenyl group, a biphenyl group, a terphenyl group, a pyrenyl group, a perylenyl group and a triphenylenyl group,
the optionally substituted heteroaryl group includes a sulfur-containing heteroaryl group; an oxygen-containing heteroaryl group; a nitrogen-containing heteroaryl group; and a heteroaryl group containing two or more heteroatoms,
the aromatic compound (2-5) having a thiol group contains, as an aromatic group, one selected from an optionally substituted aryl group and an optionally substituted heteroaryl group,
the optionally substituted aryl group includes a phenyl group, a naphthyl group, an anthracenyl group, a phenanthrenyl group, a biphenyl group, a terphenyl group, a pyrenyl group, a perylenyl group and a triphenylenyl group, and
the optionally substituted heteroaryl group includes a sulfur-containing heteroaryl group; an oxygen-containing heteroaryl group; a nitrogen-containing heteroaryl group; and a heteroaryl group containing two or more heteroatoms.

5. The method for producing a mono-cross-coupled aromatic compound (3-1) according to claim 3, which is obtained by the reaction of the aromatic compound (1) having at least two leaving groups with the aromatic boronic acid (2-1), the aromatic amino compound (2-2), the diboronic acid ester (2-3), the aromatic compound (2-4) having a hydroxyl group or the aromatic compound (2-5) having a thiol group, wherein the mono-cross-coupled aromatic compound (3-1) is selected from mono-cross-coupled aromatic compounds represented by the following general formulas (III-1-1) to (III-1-5):
X_{n - 1} -A¹ -A² : III-1-1
X_{n - 1} -A¹ -N(A³)A⁴ : III-1-2
X_{n - 1} -A¹ -B(OR³)OR⁴ : III-1-3
X_{n - 1} -A¹ -O-(Cₘ H_{2 m} )-A⁵ : 111-1-4
X_{n - 1} -A¹ -S-(Cp H_{2 p} )-A⁶ : III-1-5
wherein A¹ to A⁶, R⁵ to R⁶, n, m, p and X are the same as defined in general formulas (I) and (11-1) to (II-5).

6. The method for producing a mono-cross-coupled aromatic compound (3-1) according to any one of claims 1 to 5, wherein a melting point of the mono-cross-coupled aromatic compound (3-1) is higher than that of the aromatic compound (1) having at least two leaving groups, and a difference between the melting point of the aromatic compound (1) having at least two leaving groups and that of the mono-cross-coupled aromatic compound (3-1) is 10°C or higher.

7. The method for producing a mono-cross-coupled aromatic compound (3-1) according to any one of claims 1 to 6, wherein a melting point of the mono-cross-coupled aromatic compound (3-1) is higher than that of the aromatic compound (1) having at least two leaving groups, and a reaction temperature of the cross-coupling reaction is between the melting point of the mono-cross-coupled aromatic compound (3-1) and that of the aromatic compound (1) having at least two leaving groups.

8. The method for producing a mono-cross-coupled aromatic compound (3-1) according to any one of claims 1 to 7, wherein, regarding a di-cross-coupled aromatic compound (3-2) which underwent two cross-coupling reactions and is produced simultaneously when the aromatic compound (3-1) having at least two leaving groups is obtained, a ratio of the mono-cross-coupled aromatic compound (3-1) to the di-cross-coupled aromatic compound (3-2) (mono-cross-coupled aromatic compound (3-1)/di-cross-coupled aromatic compound (3-2)) is more than 1.

9. A method for producing a di-cross-coupled aromatic compound (3-2) which underwent two cross-coupling reactions, the method comprising:
including the method for producing a mono-cross-coupled aromatic compound (3-1) according to any one of claims 1 to 8, and
allowing the mono-cross-coupled aromatic compound (3-1) to undergo a second cross-coupling reaction.

10. The method for producing a di-cross-coupled aromatic compound (3-2) which underwent two cross-coupling reactions according to claim 9, the method comprising allowing the mono-cross-coupled aromatic compound (3-1) to undergo a second cross-coupling reaction in the absence of a solvent.

11. The method for producing a di-cross-coupled aromatic compound (3-2) according to claim 9 or 10, wherein the compound (2) used for the first cross-coupling reaction is different from the compound (2) used for the second cross-coupling reaction.

12. The method for producing a di-cross-coupled aromatic compound (3-2) according to any one of claims 9 to 11, wherein the mono-cross-coupled aromatic compound (3-1) directly undergoes the second cross-coupling reaction without purification.
